# EUROPEAN PATENT SPECIFICATION

(11) **EP 3 462 935 B1**
(45) Date of publication and mention of the grant of the patent: **26.08.2020**
(21) Application number: 17725643.5
(22) Date of filing: 30.05.2017
(51) Int. Cl.: A24F 47/00, A61M 15/06, A61M 11/04

(54) **AEROSOL-GENERATING SYSTEM COMPRISING A HEATED AEROSOL-GENERATING ARTICLE**
AEROSOLERZEUGUNGSSYSTEM MIT EINEM ERWÄRMTEN AEROSOLERZEUGUNGSARTIKEL
SYSTÈME DE GÉNÉRATION D'AÉROSOL COMPRENANT UN ARTICLE DE GÉNÉRATION D'AÉROSOL CHAUFFÉ

(30) Priority: 31.05.2016 EP 16172287; 31.05.2016 EP 16172262; 31.05.2016 EP 16172263; 31.05.2016 EP 16172265
(43) Date of publication of application: 10.04.2019
(73) Proprietor: Philip Morris Products S.A., 2000 Neuchâtel (CH)
(72) Inventor: THORENS, Michel, 1510 Moudon (CH)
(74) Representative: Millburn, Julie Elizabeth
(86) International application number: PCT/EP2017/063061
(87) International publication number: WO 2017/207586

(56) References cited:
- EP-A1- 2 046 155
- WO-A1-2015/176898
- WO-A2-2015/040180
- US-A1- 2015 027 477
- US-A1- 2016 029 694

## Description

The present invention relates to an aerosol-generating system comprising a heated aerosol-generating article and an electrically operated aerosol-generating device. The heated aerosol-generating device may comprise a liquid aerosol-forming substrate and a liquid retention medium. The present invention also relates to a consumable heated aerosol-generating article for use with the aerosol-generating device.

Recent years have seen the emergence of two main categories of heated aerosol-generating systems that produce an inhalable aerosol by heating rather than by burning an aerosol-forming substrate. One system, which may be described as an e-cigarette system, typically comprises a liquid aerosol-forming substrate contained within a cartridge of an atomiser unit. On operation, liquid is conveyed from the cartridge by a wick and is vaporised by a heating coil. A second system, which may be described as a heated tobacco system, involves the heating of a solid substrate comprising modified tobacco to produce an inhalable aerosol.

There are advantages and disadvantages of both main categories of heated aerosol-generating systems. One disadvantage of the e-cigarette system is that direct heating of the liquid substrate with a heating coil risks overheating of the liquid, particularly when the cartridge is near to empty. A further issue with a typical e-cigarette system is that the device may be used by multiple users and come into contact with many external contaminants. This provides a potential hygiene issue.

A heated tobacco system using a consumable article containing a solid aerosol-forming substrate may produce a sensorially more acceptable aerosol and does not have the same hygiene problems associated with e-cigarettes. A user may desire the wider variety of flavours that are possible with a liquid-based heated e-cigarette system, however.

One type of aerosol-generating system is an electrically operated aerosol-generating system. Known handheld electrically operated aerosol-generating systems typically comprise an aerosol-generating device comprising a battery, control electronics and an electric heater for heating an aerosol-generating article designed specifically for use with the aerosol-generating device. In some examples, the aerosol-generating article comprises an aerosol-forming substrate, such as a tobacco rod or a tobacco plug, and the heater contained within the aerosol-generating device is inserted into or around the aerosol-forming substrate when the aerosol-generating article is inserted into the aerosol-generating device.

EP 2 046 155 B1 discloses a volatilization device including a reusable heat delivery component and a volatilization component which is usable once only and is intended to be disposed of after use. The heat delivery component includes a heat pipe that extends into a heat sink at one end, and into the volatilization component at the other end so as to deliver heat from the heat sink to the volatilization component.

In existing systems, it may be difficult to evenly heat the aerosol-forming substrate with the electric heater. This may lead to some areas of the aerosol-forming substrate being overheated and may lead to some areas of the aerosol-forming substrate being under-heated. Both may make it difficult to maintain consistent aerosol characteristics. This may be a particular issue with aerosol-generating articles in which the aerosol-forming substrate is a liquid aerosol-forming substrate, since depletion of the aerosol-forming substrate may cause one or more parts of the aerosol-generating article to overheat.

It would be desirable to provide means for mitigating the above described problems.

According to a first aspect of the present invention there is provided an electrically operated aerosol-generating system comprising an aerosol-generating article and an electrically operated aerosol-generating device. The aerosol-generating article comprises an aerosol-forming substrate and has a mouth end and a distal end upstream from the mouth end. The electrically operated aerosol-generating device comprises a housing having a cavity configured to receive the distal end of the aerosol-generating article. The aerosol-generating system further comprises an electric heating element and a heat diffuser comprising a non-combustible porous body for absorbing heat from the electric heating element such that, in use, air drawn through the aerosol-generating article from the distal end to the mouth end is heated by the heat absorbed in the porous body.

As used herein, the term "heated aerosol-generating article" refers to an article comprising an aerosol-generating substrate that, when heated, releases volatile compounds that can form an aerosol. A "heated aerosol-generating article" refers to an article comprising an aerosol-forming substrate that is intended to be heated rather than combusted in order to release volatile compounds that can form an aerosol. The aerosol formed by heating the aerosol-forming substrate may contain fewer known harmful constituents than would be produced by combustion or pyrolytic degradation of the aerosol-forming substrate. The aerosol-generating article is preferably removably couplable to an aerosol-generating device. The article may be disposable or reusable.

As used herein, the term "aerosol-generating device" is a device that engages or interacts with a heated aerosol-generating article to form an inhalable aerosol. The device that interacts with an aerosol-forming substrate to generate an aerosol. An electrically operated aerosol-generating device is a device comprising one or more components used to supply energy from a power supply, e.g. an electrical power supply, to heat an aerosol-forming substrate to generate an aerosol. The aerosol-generating device may be an electrically operated aerosol-generating device comprising a heating means that is operated by electrical power to heat an aerosol-forming substrate of an aerosol-generating article to generate an aerosol. The heating means may be a heater for heating air supplied to an aerosol-forming substrate. The heating means may be an inductor for heating a susceptor, for example to heat air supplied to an aerosol-forming substrate.

An aerosol-generating device may be described as a heated aerosol-generating device, which is an aerosol-generating device comprising a heating element. The heating element or heater is used to heat an aerosol-forming substrate of an aerosol-generating article to generate an aerosol, or the solvent-evolving substrate of a cleaning consumable to form a cleaning solvent. An aerosol-generating device may be an electrically heated aerosol-generating device, which is an aerosol-generating device comprising a heating element that is operated by electrical power to heat an aerosol-forming substrate of an aerosol-generating article to generate an aerosol.

The electric heating element may form part of the aerosol-generating article, part of the heat diffuser, part of the aerosol-generating device, or any combination thereof. In preferred embodiments, the electric heating element forms part of the device.

In some embodiments, the heat diffuser may form part of the aerosol-generating article or the aerosol-generating device. In preferred embodiments, the heat diffuser is removably couplable to the aerosol-generating device. In such embodiments, the heat diffuser may form part of the aerosol-generating article or may be an independent component that does not form part of the aerosol-generating article or the aerosol-generating device. The heat diffuser may be removably couplable to the aerosol-generating article and the aerosol-generating device. The device may be configured to removably couple to the heat diffuser. The cavity of the device may be configured to removably receive the heat diffuser. The electric heating element may be configured to thermally couple with the heat diffuser when the heat diffuser is removably coupled to the aerosol-generating device. The electric heating element may be configured to thermally couple with the heat diffuser when the heat diffuser is received in the cavity of the aerosol-generating device.

As used herein, the term removably couplable' is used to mean that two or more components of the system, such as the heat diffuser and the device, or the article and the device can be coupled and uncoupled from one another without significantly damaging either component. For example, the article may be removed from the device when the aerosol-forming substrate has been consumed. The heat diffuser may be disposable. The heat diffuser may be reusable.

The heat diffuser may be a removable component of the aerosol-generating system. For example, heat diffuser may be in the form of a removably couplable component that engages with the aerosol generating device to alter the manner in which the aerosol generating device heats aerosol generating articles. As an example, the aerosol generating device may comprise an insertable heating element for insertion into a solid aerosol forming substrate of a heated aerosol-generating article. The heating element contacts the solid aerosol-forming substrate and heats it to generate an aerosol. The heat diffuser may be configured to engage with the insertable heating element. The heat diffuser may then be heated by the heating element and heat air that passes through the heating element. The heated air may then volatilize an aerosol-forming substrate of a heated aerosol-generating article that is located downstream of the heat diffusor. In this way the manner in which the aerosol-generating device heats an aerosol-forming substrate may be changed from direct contact to indirect heating of air. The same aerosol-generating device may then be used to heat different types of aerosol-generating article, thereby providing a greater choice to the user.

In preferred embodiments, the system is configured such that either: when the heat diffuser is coupled to the aerosol-generating device, the heat diffuser absorbs heat from the electric heating element and air drawn through the aerosol-generating article from the distal end to the mouth end is heated by the heat absorbed in the porous body of the heat diffuser; or when the heat diffuser is not coupled to the aerosol-generating device, the aerosol-generating article absorbs heat from the electric heating element.

Advantageously, in use, the heat diffuser absorbs heat from a heating element and transfers it to air drawn through the heat diffuser so that the air can heat the aerosol-forming substrate downstream of the heat diffuser primarily by convection. This may provide more even heating of the aerosol-forming substrate relative to existing systems in which the aerosol-forming substrate is heated primarily by conduction from the heating element. For example, it may reduce or prevent areas of local high temperature, or "hot spots", from occurring in the aerosol-forming substrate that may otherwise be caused by conductive heating. This may be of particular benefit when the heat diffuser is used with aerosol-generating articles in which the aerosol-forming substrate is a liquid aerosol-forming substrate, since it may help to prevent overheating that may otherwise result from depletion of the aerosol-forming substrate. For example, where the aerosol-forming substrate comprises a liquid aerosol-forming substrate held in a liquid retention medium, the heat diffuser may help to reduce or prevent overheating of the aerosol-forming substrate or the liquid retention medium, even when the liquid retention medium is dry.

The heat diffuser may be arranged and configured to heat the air to between about 180 degrees Celsius and 250 degrees Celsius. In preferred embodiments, the heat diffuser heats the air to between about 200 degrees Celsius and 220 degrees Celsius.

A preferred system may comprise a heated aerosol-generating device, at least one heated aerosol-generating article having a liquid aerosol-forming substrate, and at least one heated aerosol-forming substrate having a solid aerosol-forming substrate, for example an aerosol-forming substrate made from homogenized tobacco material. Preferably, the system may further comprise a removably couplable heat diffusor for engagement with the aerosol-generating device to change the manner in which the aerosol-generating device provides heat to the aerosol-forming substrate.

As used herein, the term "porous" is intended to encompass materials that are inherently porous as well as substantially non-porous materials that are made porous or permeable through the provision of a plurality of holes. The porous body may be formed from a plug of porous material, for example a ceramic or metal foam. Alternatively, the porous body may be formed from a plurality of solid elements between which a plurality of apertures are provided. For example, the porous body may comprise a bundle of fibres, or a lattice of interconnected filaments. The porous material must have pores of a sufficient size that air can be drawn through the porous body through the pores. For example, the pores in the porous body may have an average transverse dimension of less than about 3.0 mm, more preferably less than about 1.0 mm, most preferably less than about 0.5 mm. Alternatively or in addition, the pores may have an average transverse dimension that is greater than about 0.01 mm. For example, the pores may have an average transverse dimension that is between about 0.01 mm and about 3.0 mm, more preferably between about 0.01 mm and about 1.0 mm, and most preferably between about 0.01 mm and about 0.5 mm.

As used herein, the term "pores" relates to regions of a porous article that are devoid of material. For example, a transverse area of porous body will comprise portions of the material forming the body and portions that are voids between the portions of material.

The average transverse dimension of the pores is calculated by taking the average of the smallest transverse dimension of each of the pores. The pore sizes may be substantially constant along the length of the porous body. Alternatively, the pore sizes may vary along the length of the porous body.

As used herein, the term "transverse dimension" refers to a dimension that is in a direction which is substantially perpendicular to the longitudinal direction of the heated aerosol-generating article, the electrically operated aerosol-generating device or the porous body.

The porosity distribution of the porous body may be substantially uniform. That is, the pores within the porous body may be distributed substantially evenly over the transverse area of the porous body. The porosity distribution may differ across the transverse area of the porous body. That is, the local porosity in one or more sub-areas of the transverse area may be greater than the local porosity in one or more other sub-areas of the transverse area. For example, the local porosity in one or more sub-areas of the transverse area may be between 5 percent and 80 percent greater than the local porosity in one or more other sub-areas of the transverse area.

As used herein, the term "transverse area" relates to an area of the porous body that is in a plane generally perpendicular to the longitudinal dimension of the porous body. For example, the porous body may be a rod and the transverse area may be a cross-section of the rod taken at any length along the rod, or the transverse area may be an end face of the rod.

As used herein, the term "porosity" refers to the volume fraction of void space in a porous article. As used herein, the term "local porosity" refers to the fraction of pores within a sub-area of the porous body.

By varying the porosity distribution, air flow through the porous body may be altered as desired, for example to provide improved aerosol characteristics. For example, this porosity distribution may be varied according to the airflow characteristics of an aerosol-generating system, or the temperature profile of a heating element, with which the heat diffuser is intended for use.

In some examples, the local porosity may be lower towards a centre portion of the porous body. With this arrangement, the air flow through the centre portion of the porous body is decreased relative to the periphery of the porous body. This may be advantageous depending on the temperature profile of the heating element or on the airflow characteristics of the aerosol-generating system with which the heat diffuser is intended for use. For example, this arrangement may be of particular benefit when used with an internal heating element positioned in use towards a central portion of the heat diffuser, since it may allow for increased heat transfer from the heating element to the porous body.

In other examples, the local porosity may be greater towards a centre portion of the porous body. This arrangement may enable increased air flow through the centre of the porous body and may be advantageous depending on the temperature profile of the heating element or on the airflow characteristics of the aerosol-generating system with which the heat diffuser is intended for use. For example, this arrangement may be of particular benefit when used with an external heating element positioned in use around the periphery of the heat diffuser, since it may allow for increased heat transfer from the heating element to the porous body.

As porous bodies have a high surface-area-to-volume ratio, the heat diffuser may allow quick and efficient heating of air drawn through the porous body. This may allow for homogenous heating of air drawn through the porous body and, consequently, more even heating of an aerosol-forming substrate downstream of the heat diffuser.

In preferred embodiments, the porous body has a surface area-to-volume ratio of at least 20 to 1, preferably at least 100 to 1, more preferably of at least 500 to 1. Advantageously, this may provide a compact heat diffuser while allowing for particularly efficient transfer of thermal energy from the heating element to air drawn through the porous body. This may lead to quicker, and homogenous heating of air drawn through the porous body and, consequently, more even heating of an aerosol-forming substrate downstream of the heat diffuser relative to porous bodies having lower surface area to volume ratios.

In preferred embodiments, the porous body has a high specific surface area. This is a measure of the total surface area of a body per unit of mass. Advantageously, this may provide a low mass heat diffuser with a large surface area for efficient transfer of thermal energy from the heating element to air drawn through the porous body. For example, the porous body may have a specific surface area of at least 0.01 m² per gram, preferably at least 0.05 m² per gram, more preferably at least 0.1 m² per gram, most preferably at least 0.5 m² per gram.

The porous body preferably has an open cell porosity of between about 60 percent to about 90 percent void volume to material volume.

In some embodiments, the porous body has a low resistance to draw. That is, the porous body may offer a low resistance to the passage of air through the heat diffuser. In such examples, the porous body does not substantially affect the resistance to draw of an aerosol-generating system with which the heat diffuser is intended for use. In some embodiments, the resistance to draw (RTD) of the porous body is between about 10 to 130 mm H₂O, preferably between about 40 to 100 mm H₂O. The RTD of a specimen refers to the static pressure difference between the two ends of the specimen when it is traversed by an air flow under steady conditions in which the volumetric flow is 17.5 millilitres per second at the output end. The RTD of a specimen can be measured using the method set out in ISO Standard 6565:2002 with any ventilation blocked.

The porous body may be formed from a heat storage material.

As used herein, the term "heat storage material" refers to a material having a high heat capacity. With this arrangement, the porous body may act as a heat reservoir, allowing the heat diffuser to absorb and store heat from the heating element and to subsequently release the heat over time to the aerosol-forming substrate, via air drawn through the porous body.

Where the porous body is formed from a heat storage material, preferably, the porous body is formed from a material having a specific heat capacity of at least 0.5 J/g.K, preferably at least 0.7 J/g.K, more preferably at least 0.8 J/g.K at 25 degrees Celsius and constant pressure. As the specific heat capacity of a material is effectively a measure of the material's ability to store thermal energy, forming the porous body from a material having a high heat capacity may allow the porous body to provide a large heat reservoir for heating air drawn through the heat diffuser without substantially increasing the weight of an aerosol-generating system with which the heat diffuser is intended for use.

The porous body may be formed from any suitable material or materials. Where the porous body is formed from a heat storage material, suitable materials include, but are not limited to, glass fibre, glass mat, ceramic, silica, alumina, carbon, and minerals, or any combination thereof.

The heat storage material may be thermally insulating. As used herein, the term "thermally insulating" refers to a material having a thermal conductivity of less than 100 W/m.K, preferably less than 40 W/m.K, or less than 10 W/m.K at 23 degrees Celsius and a relative humidity of 50%. This may result in a heat diffuser with a higher thermal inertia relative to thermally conductive heat diffusers to reduce variations in the temperature of air drawn through the porous body caused by temperature fluctuations in the heating element. This may result in more consistent aerosol characteristics.

The porous body may be thermally conductive. As used herein, the term "thermally conductive" refers to a material having a thermal conductivity of at least 10 W/m.K, preferably at least 40 W/m.K, more preferably at least 100 W/m.K at 23 degrees Celsius and a relative humidity of 50%. Where the porous body is thermally conductive, preferably, the porous body is formed from a material having a thermal conductivity of at least 40 W/m.K, preferably at least 100 W/m.K, more preferably at least 150 W/m.K, and most preferably at least 200 W/m.K at 23 degrees Celsius and a relative humidity of 50%.

Advantageously, this may reduce the thermal inertia of the heat diffuser and allow the temperature of the heat diffuser to quickly adjust to changes in the temperature of the heating element, for example where the heating element is heated according to a heating regime which changes over time, while still allowing the air drawn through the porous body to be evenly heated. Further, by having a high thermal conductivity, the thermal resistance through the porous body will be lower. This may allow the temperature of portions of the porous body which are remote from the heating element in use to be at a similarly high temperature as the portions of the porous body which are closest to the heating element in use. This may provide for particularly efficient heating of air drawn through the porous body.

Where the porous body is thermally conductive, preferably, the porous body is formed from a material having a thermal conductivity of at least 40 W/m.K, preferably at least 100 W/m.K, more preferably at least 150 W/m.K, most preferably at least 200 W/m.K at 23 degrees Celsius and a relative humidity of 50%.

Where the porous body is thermally conductive, suitable thermally conductive materials include, but are not limited to, aluminium, copper, zinc, steel, silver, thermally conductive polymers, or any combination or alloy thereof.

In some embodiments, the porous body is formed from a heat storage material which is also thermally conductive, such as aluminium.

In embodiments in which the heat diffuser does not form part of the aerosol-generating device, the porous body may be configured to be penetrated by an electric heating element forming part of an aerosol-generating device when the heat diffuser is coupled to the aerosol-generating device. The term "penetrated" is used to mean that the heating element at least partially extends into the porous body. Thus, the heating element may be sheathed within the porous body. With this arrangement, by the act of penetration, the heating element is brought into close proximity to, or contact with, the porous body. This may increase heat transfer between the heating element and the porous body and, consequently, to air drawn through the porous body relative to examples in which the porous body is not penetrated by the heating element.

The heating element may conveniently be shaped as a needle, pin, rod, or blade that may be inserted into the heat diffuser. The aerosol-generating device may comprise more than one heating element and in this description reference to a heating element means one or more heating elements.

The porous body may define a cavity or hole for receiving the electric heating element when the heat diffuser is coupled to the aerosol-generating device.

In any of the above embodiments, the porous body may be rigid.

The porous body may be pierceable by the heating element when the heat diffuser is coupled to the aerosol-generating device. For example, the porous body may comprise a foam, such as a polymer, metal or ceramic foam, that is pierceable by the heating element.

In any of the above embodiments, the electric heating element may be provided as part of an aerosol-generating device with which the heat diffuser is intended for use, or as part of the aerosol-generating article with which the heat diffuser is intended for use, as part of the heat diffuser, or any combination thereof. The electric heating element may be coupled to the porous body of the heat diffuser. The heat diffuser may comprise an electric heating element thermally coupled to the porous body. In such embodiments, the porous body is arranged to absorb heat from the heating element and transfer it to air drawn through the porous body. With this arrangement, the heating element can be easily replaced by replacing the heat diffuser, while allowing the aerosol-generating device to be reused with a new heat diffuser.

The electric heating element may comprise one or more external heating elements, one or more internal heating elements, or one or more external heating elements and one or more internal heating elements. As used herein, the term "external heating element" refers to a heating element that is positioned outside of the heat diffuser when an aerosol-generating system comprising the heat diffuser is assembled. As used herein, the term "internal heating element" refers to a heating element that is positioned at least partially within the heat diffuser when an aerosol-generating system comprising the heat diffuser is assembled. The electric heating element may be embedded in the porous body of the heat diffuser.

The one or more external heating elements may comprise an array of external heating elements arranged around the periphery of the heat diffuser, for example on the outer surface of the porous body. In certain examples, the external heating elements extend along the longitudinal direction of the heat diffuser. With this arrangement, the heating elements may extend along the same direction in which the heat diffuser may be inserted into and removed from a cavity in an aerosol-generating device. This may reduce interference between the heating elements and the aerosol-generating device relative to devices in which the heating elements are not aligned with the length of the heat diffuser. In some embodiments, the external heating elements extend along the length direction of the heat diffuser and are spaced apart in the circumferential direction. Where the heating element comprises one or more internal heating elements, the one or more internal heating elements may comprise any suitable number of heating elements. For example, the heating element may comprise a single internal heating element. The single internal heating element may extend along the longitudinal direction of the heat diffuser.

Where the electric heating element forms part of the heat diffuser, the heat diffuser may further comprise one or more electrical contacts by which the electric heating element is connectable to a power supply, for example a power supply in the aerosol-generating device.

The electric heating element may be an electrically resistive heating element.

The electric heating element may comprise a susceptor in thermal contact with the porous body. The electric heating element may be a susceptor forming part of the heat diffuser. Preferably, the susceptor is embedded in the porous body.

As used herein, the term 'susceptor' refers to a material that can convert electromagnetic energy into heat. When located within a fluctuating electromagnetic field, eddy currents induced in the susceptor cause heating of the susceptor. As the susceptor is in thermal contact with the heat diffuser, the heat diffuser is heated by the susceptor.

In such embodiments, the heat diffuser is designed to engage with an electrically-operated aerosol-generating device comprising an induction heating source. The induction heating source, or inductor, generates the fluctuating electromagnetic field for heating a susceptor located within the fluctuating electromagnetic field. In use, the heat diffuser engages with the aerosol-generating device such that the susceptor is located within the fluctuating electromagnetic field generated by the inductor.

The susceptor may be in the form of a pin, rod, or blade. The susceptor preferably has a length of between 5 mm and 15 mm, for example between 6 mm and 12 mm, or between 8 mm and 10 mm. The susceptor preferably has a width of between 1 mm and 5 mm and may have a thickness of between 0.01 mm and 2 mm. for example between 0.5 mm and 2 mm. A preferred embodiment of susceptor may have a thickness of between 10 micrometres and 500 micrometres, or even more preferably between 10 and 100 micrometres. If the susceptor has a constant cross-section, for example a circular cross-section, it has a preferable width or diameter of between 1 mm and 5 mm.

The susceptor may be formed from any material that can be inductively heated to a temperature sufficient to generate an aerosol from the aerosol-forming substrate downstream of the heat diffuser. Preferred susceptors comprise a metal or carbon. A preferred susceptor may comprise a ferromagnetic material, for example ferritic iron, or a ferromagnetic steel or stainless steel. A suitable susceptor may be, or comprise, aluminium. Preferred susceptors may be formed from 400 series stainless steels, for example grade 410, or grade 420, or grade 430 stainless steel. Different materials will dissipate different amounts of energy when positioned within electromagnetic fields having similar values of frequency and field strength. Thus, parameters of the susceptor such as material type, length, width, and thickness may all be altered to provide a desired power dissipation within a known electromagnetic field.

Preferred susceptors may be heated to a temperature in excess of 250 degrees Centigrade. Suitable susceptors may comprise a non-metallic core with a metal layer disposed on the non-metallic core, for example metallic tracks formed on a surface of a ceramic core.

A susceptor may have a protective external layer, for example a protective ceramic layer or protective glass layer encapsulating the susceptor. The susceptor may comprise a protective coating formed by a glass, a ceramic, or an inert metal, formed over a core of the susceptor.

The heat diffuser may contain a single susceptor. Alternatively, the heat diffuser may comprise more than one susceptor.

Heat diffusers according to the invention may comprising a piercing member at one end of the porous body. This may allow the heat diffuser to conveniently and easily pierce a seal at an end of an aerosol-generating article with which it is intended for use when the heat diffuser is engaged with the aerosol-generating article. Where the aerosol-generating article with which the heat diffuser is intended for use comprises a frangible capsule, for example a frangible capsule containing an aerosol-forming substrate, the piercing member may allow the heat diffuser to conveniently and easily pierce the frangible capsule when the heat diffuser is engaged with the aerosol-generating article.

The downstream end of the piercing member preferably has a cross-sectional area that is smaller than the cross-sectional area of the region of the piercing member immediately upstream of the downstream end. In a particularly preferred embodiment, the cross-sectional area of the piercing member narrows towards a tapered tip at its downstream end.

The piercing member may be formed by the porous body. Alternatively, the piercing member may be a separate component attached at the downstream end of the porous body.

The aerosol-forming substrate may be a solid aerosol-forming substrate. Alternatively, the aerosol-forming substrate may comprise both solid and liquid components. The aerosol-forming substrate may comprise tobacco. The aerosol-forming substrate may comprise a tobacco-containing material containing volatile tobacco flavour compounds which are released from the substrate upon heating. The aerosol-forming substrate may comprise a non-tobacco material. The aerosol-forming substrate may comprise tobacco-containing material and non-tobacco containing material.

The aerosol-forming substrate may further comprise an aerosol former that facilitates the formation of a dense and stable aerosol. Examples of suitable aerosol formers are glycerine and propylene glycol.

The aerosol-forming substrate may comprise a solid aerosol-forming substrate. The aerosol-forming substrate may comprise a tobacco-containing material containing volatile tobacco flavour compounds which are released from the substrate upon heating. The aerosol-forming substrate may comprise a non-tobacco material..

The aerosol-forming substrate may include at least one aerosol former. As used herein, the term 'aerosol former' is used to describe any suitable known compound or mixture of compounds that, in use, facilitates formation of an aerosol. Suitable aerosol formers are substantially resistant to thermal degradation at the operating temperature of the aerosol-generating article. Examples of suitable aerosol formers are glycerine and propylene glycol. Suitable aerosol formers include, but are not limited to: polyhydric alcohols, such as propylene glycol, triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Preferred aerosol formers are polyhydric alcohols or mixtures thereof, such as propylene glycol, triethylene glycol, 1,3-butanediol and, most preferred, glycerine. The aerosol-forming substrate may comprise a single aerosol former. Alternatively, the aerosol-forming substrate may comprise a combination of two or more aerosol formers. The aerosol-forming substrate may have an aerosol former content of greater than 5 percent on a dry weight basis. The aerosol-forming substrate may have an aerosol former content of between approximately 5 percent and approximately 30 percent on a dry weight basis. The aerosol-forming substrate may have an aerosol former content of approximately 20 percent on a dry weight basis.

The aerosol-forming substrate may comprise a liquid aerosol-forming substrate. The liquid aerosol-forming substrate may comprise a nicotine solution. The liquid aerosol-forming substrate preferably comprises a tobacco-containing material comprising volatile tobacco flavour compounds which are released from the liquid upon heating. The liquid aerosol-forming substrate may comprise a non-tobacco material. The liquid aerosol-forming substrate may include water, solvents, ethanol, plant extracts and natural or artificial flavours. Preferably, the liquid aerosol-forming substrate further comprises an aerosol former.

In some embodiments, the aerosol-generating article comprises a liquid aerosol-forming substrate and a liquid retention medium for retaining the liquid aerosol-forming substrate.

The liquid retention medium preferably comprises an absorbent material, for example an absorbent polymeric material. Examples of suitable liquid retention materials include fibrous polymers and porous polymers such as open-cell foams. The liquid retention medium may comprise a fibrous cellulose acetate or a fibrous cellulose polymer. The liquid retention medium may comprise a porous polypropylene material. Suitable materials capable of retaining a liquid will be known to the skilled person.

The liquid retention medium is either located within an air-flow path through the heated aerosol-generating article or defines at least a portion of an air-flow path through the aerosol-generating article. Preferably, one or more holes defined through the liquid retention medium define a portion of the air-flow path through the heated aerosol-generating article between the distal end of the article and the mouth end of the article.

The liquid retention medium may be in the form of a tube having a central lumen. Walls of the tube would then be formed from, or comprise, a suitable liquid-retention material.

The liquid aerosol-forming substrate be incorporated into the liquid retention medium immediately prior to use. For example, a dose of liquid aerosol-forming substrate may be injected into the liquid retention medium immediately prior to use.

Articles according to the invention may comprise a liquid aerosol-forming substrate contained within a frangible capsule. The frangible capsule is described in greater detail below. In preferred embodiments, the aerosol-forming substrate is a liquid aerosol-forming substrate and the article further comprises a frangible capsule containing the liquid aerosol-forming substrate, and a liquid retention medium downstream of the heat diffuser and arranged to absorb the liquid aerosol-forming substrate when the frangible capsule is broken.

The frangible capsule may be located within the porous carrier material. For example, the porous carrier material may be provided in the form of a liquid retention tube and the frangible capsule is located within the lumen of the tube.

The frangible capsule may be located adjacent to the liquid retention medium within the article such that the liquid-aerosol-forming substrate released from the frangible capsule can contact and be retained by the liquid retention medium. The frangible capsule may be located within the liquid retention medium. For example, the liquid retention medium may comprise a plug of material in which the capsule is embedded. Preferably, article comprises a tubular liquid retention medium and the frangible capsule containing the liquid aerosol-forming substrate is located within the lumen of the tubular liquid retention medium.

Where the aerosol-forming substrate is a solid aerosol-forming substrate, the solid aerosol-forming substrate may be immediately downstream of the heat diffuser. For example, the solid aerosol-forming substrate may abut the heat diffuser. In other embodiments, the solid aerosol-forming substrate may be spaced apart in the longitudinal direction from the heat diffuser.

In certain preferred embodiments, the aerosol-forming substrate is a liquid aerosol-forming substrate and the article further comprises a liquid retention medium for retaining the liquid aerosol-forming substrate. In such embodiments, the liquid retention medium may be immediately downstream of the heat diffuser. For example, the liquid retention medium may abut the heat diffuser. In other embodiments, the liquid retention medium may be spaced apart in the longitudinal direction from the heat diffuser.

In one particular embodiment, the aerosol-forming substrate is a liquid aerosol-forming substrate and the article further comprises a liquid retention medium for retaining the liquid aerosol-forming substrate, the liquid retention medium being spaced apart in the longitudinal direction from the heat diffuser.

With this arrangement, conductive heat transfer between the heat diffuser and the liquid retention medium, or a solid aerosol-forming substrate, may be reduced. This may further reduce or prevent areas of local high temperature, or "hot spots", from occurring in the liquid retention medium, or the aerosol-forming substrate, that may otherwise be caused by conductive heating.

Aerosol-generating articles according to the present invention may further comprise a support element may be located immediately downstream of the aerosol-forming substrate or, where the article comprises a liquid retention medium for retaining a liquid aerosol-forming substrate, immediately downstream of the liquid retention medium. The support element may abut the aerosol-forming substrate or the liquid retention medium.

The support element may be formed from any suitable material or combination of materials. For example, the support element may be formed from one or more materials selected from the group consisting of: cellulose acetate; cardboard; crimped paper, such as crimped heat resistant paper or crimped parchment paper; and polymeric materials, such as low density polyethylene (LDPE). In a preferred embodiment, the support element is formed from cellulose acetate. The support element may comprise a hollow tubular element. For example, the support element comprises a hollow cellulose acetate tube. The support element preferably has an external diameter that is approximately equal to the external diameter of the aerosol-generating article.

The support element may have an external diameter of between approximately 5 millimetres and approximately 12 millimetres, for example of between approximately 5 millimetres and approximately 10 millimetres or of between approximately 6 millimetres and approximately 8 millimetres. For example, the support element may have an external diameter of 7.2 millimetres +/- 10 percent.

The support element may have a length of between approximately 5 millimetres and approximately 15 mm. In a preferred embodiment, the support element has a length of approximately 8 millimetres.

An aerosol-cooling element may be located downstream of the aerosol-forming substrate, for example an aerosol-cooling element may be located immediately downstream of a support element, and may abut the support element. The aerosol-cooling element may be located immediately downstream of the aerosol-forming substrate or, where the article comprises a liquid retention medium for retaining a liquid aerosol-forming substrate, immediately downstream of the liquid retention medium. For example, the aerosol-cooling element may abut the aerosol-forming substrate or the liquid retention medium.

The aerosol-cooling element may have a total surface area of between approximately 300 square millimetres per millimetre length and approximately 1000 square millimetres per millimetre length. In a preferred embodiment, the aerosol-cooling element has a total surface area of approximately 500 square millimetres per millimetre length.

The aerosol-cooling element preferably has a low resistance to draw. That is, the aerosol-cooling element preferably offers a low resistance to the passage of air through the aerosol-generating article. Preferably, the aerosol-cooling element does not substantially affect the resistance to draw of the aerosol-generating article.

The aerosol-cooling element may comprise a plurality of longitudinally extending channels. The plurality of longitudinally extending channels may be defined by a sheet material that has been one or more of crimped, pleated, gathered and folded to form the channels. The plurality of longitudinally extending channels may be defined by a single sheet that has been one or more of crimped, pleated, gathered and folded to form multiple channels. Alternatively, the plurality of longitudinally extending channels may be defined by multiple sheets that have been one or more of crimped, pleated, gathered and folded to form multiple channels.

In some embodiments, the aerosol-cooling element may comprise a gathered sheet of material selected from the group consisting of metallic foil, polymeric material, and substantially non-porous paper or cardboard. In some embodiments, the aerosol-cooling element may comprise a gathered sheet of material selected from the group consisting of polyethylene (PE), polypropylene (PP), polyvinylchloride (PVC), polyethylene terephthalate (PET), polylactic acid (PLA), cellulose acetate (CA), and aluminium foil.

In a preferred embodiment, the aerosol-cooling element comprises a gathered sheet of biodegradable material. For example, a gathered sheet of non-porous paper or a gathered sheet of biodegradable polymeric material, such as polylactic acid or a grade of Mater-Bi® (a commercially available family of starch based copolyesters). In a particularly preferred embodiment, the aerosol-cooling element comprises a gathered sheet of polylactic acid.

The aerosol-cooling element may be formed from a gathered sheet of material having a specific surface area of between approximately 10 square millimetres per milligram and approximately 100 square millimetres per milligram weight. In some embodiments, the aerosol-cooling element may be formed from a gathered sheet of material having a specific surface area of approximately 35 mm²/mg.

In use, the aerosol-cooling element may be arranged and configured to cool the heated air and vapour drawn through the element to between about 80 degrees Celsius and 120 degrees Celcius. In preferred embodiments, the aerosol-cooling element cools the heated air and vapour drawn through the element to about 100 degrees Celsius.

The aerosol-generating article may comprise a mouthpiece located at the mouth end of the aerosol-generating article. The mouthpiece may be located immediately downstream of an aerosol-cooling element and may abut the aerosol-cooling element. The mouthpiece may be located immediately downstream of the aerosol-forming substrate or, where the article comprises a liquid retention medium for retaining a liquid aerosol-forming substrate, immediately downstream of the liquid retention medium. In such embodiments, the mouthpiece may abut the aerosol-forming substrate, or the liquid retention medium. The mouthpiece may comprise a filter. The filter may be formed from one or more suitable filtration materials. Many such filtration materials are known in the art. In one embodiment, the mouthpiece may comprise a filter formed from cellulose acetate tow.

The mouthpiece preferably has an external diameter that is approximately equal to the external diameter of the aerosol-generating article. The mouthpiece may have an external diameter of a diameter of between approximately 5 millimetres and approximately 10 millimetres, for example of between approximately 6 millimetres and approximately 8 millimetres. In a preferred embodiment, the mouthpiece has an external diameter of 7.2 millimetres +/- 10%.

The mouthpiece may have a length of between approximately 5 millimetres and approximately 20 millimetres. For example, the mouthpiece may have a length of from about 7 mm to about 12 mm.

The elements of the aerosol-forming article may be circumscribed by an outer wrapper, for example in the form of a rod. The wrapper may circumscribe at least a downstream portion of the heat diffuser. In some embodiments, the wrapper circumscribes the heat diffuser along substantially the entire length of the heat diffuser. The outer wrapper may be formed from any suitable material or combination of materials. The outer wrapper may be non-porous. The outer wrapper may be liquid-impervious.

The aerosol-generating article may be substantially cylindrical in shape. The aerosol-generating article may be substantially elongate. The aerosol-generating article may have a length and a circumference substantially perpendicular to the length. The aerosol-forming substrate or a porous carrier material in which the aerosol-forming substrate is absorbed during use, may be substantially cylindrical in shape. The aerosol-forming substrate or the porous carrier material may be substantially elongate. The aerosol-forming substrate, or the porous carrier material, may also have a length and a circumference substantially perpendicular to the length.

The aerosol-forming substrate or, where applicable, the liquid retention medium, may have a length of between about 7 mm and about 15 mm. In one embodiment, the aerosol-forming substrate, or the liquid retention medium, may have a length of approximately 10 mm. Alternatively, the aerosol-forming substrate, or the liquid retention medium, may have a length of approximately 12 mm.

The aerosol-generating substrate or liquid retention medium, preferably has an external diameter that is approximately equal to the external diameter of the aerosol-generating article. The external diameter of the aerosol-forming substrate, or the liquid retention medium, may be between approximately 5 mm and approximately 12 mm. In one embodiment, the aerosol-forming substrate, or the liquid retention medium, may have an external diameter of approximately 7.2 mm +/- 10 percent.

A heated aerosol-generating system may be provided comprising an aerosol-generating device and a heated aerosol-generating article according to any of the embodiments discussed above. Preferably the aerosol-generating device is an electrically operated aerosol-generating device.

An aerosol-generating device may be described as a heated aerosol-generating device, which is an aerosol-generating device comprising a heating element or heater. The heating element or heater is used to heat an aerosol-forming substrate of an aerosol-generating article to generate an aerosol.

The aerosol-generating device may be an electrically heated aerosol-generating device, which is an aerosol-generating device comprising a heating element that is operated by electrical power to heat an aerosol-forming substrate of an aerosol-generating article to generate an aerosol.

The aerosol-generating device may comprise electric circuitry configured to control the supply of power from a power supply to an electric heating element of the system.

The aerosol-generating device of the aerosol-generating system may comprise: a housing having a cavity for receiving the heated aerosol-generating article and a controller configured to control the supply of power from a power supply to an electric heating element of the system.

The electric heating element may comprise one or more heating elements.

In some embodiments, the electrically operated aerosol-generating device comprises an electric heating element and a housing having a cavity, and wherein the heated aerosol-generating article is received in the cavity. The heating element may conveniently be shaped as a needle, pin, rod, or blade that may be inserted into the article.

Aerosol-generating systems according to the invention include an electric heating element. The electric heating element may comprise one or more external heating elements, one or more internal heating elements, or one or more external heating elements and one or more internal heating elements. As used herein, the term "external heating element" refers to a heating element that is positioned outside of the heat diffuser when an aerosol-generating system comprising the heat diffuser is assembled. As used herein, the term "internal heating element" refers to a heating element that is positioned at least partially within the heat diffuser when an aerosol-generating system comprising the heat diffuser is assembled.

The one or more external heating elements may comprise an array of external heating elements arranged around the inner surface of the cavity. In certain examples, the external heating elements extend along the longitudinal direction of the cavity. With this arrangement, the heating elements may extend along the same direction in which the heat diffuser and the article are inserted into and removed from the cavity. This may reduce interference between the heating elements and the heat diffuser relative to devices in which the heating elements are not aligned with the length of the cavity. In some embodiments, the external heating elements extend along the length direction of the cavity and are spaced apart in the circumferential direction. Where the heating element comprises one or more internal heating elements, the one or more internal heating elements may comprise any suitable number of heating elements. For example, the heating element may comprise a single internal heating element. The single internal heating element may extend along the longitudinal direction of the cavity.

The electric heating element may comprise an electrically resistive material. Suitable electrically resistive materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, Constantan, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. Timetal® is a registered trade mark of Titanium Metals Corporation, 1999 Broadway Suite 4300, Denver Colorado. In composite materials, the electrically resistive material may optionally be embedded in, encapsulated or coated with an insulating material or vice-versa, depending on the kinetics of energy transfer and the external physicochemical properties required. The heating element may comprise a metallic etched foil insulated between two layers of an inert material. In that case, the inert material may comprise Kapton®, all-polyimide or mica foil. Kapton® is a registered trade mark of E.I. du Pont de Nemours and Company, 1007 Market Street, Wilmington, Delaware 19898, United States of America.

Where the electric heating element comprises a susceptor in thermal contact with the porous body of the heat diffuser, the aerosol-generating device preferably comprises an inductor arranged to generate a fluctuating electromagnetic field within the cavity and an electrical power supply connected to the inductor. The inductor may comprise one or more coils that generate a fluctuating electromagnetic field. The coil or coils may surround the cavity.

Preferably the device is capable of generating a fluctuating electromagnetic field of between 1 and 30 MHz, for example, between 2 and 10 MHz, for example between 5 and 7 MHz. Preferably the device is capable of generating a fluctuating electromagnetic field having a field strength (H-field) of between 1 and 5 kA/m, for example between 2 and 3 kA/m, for example about 2.5 kA/m.

Preferably, the aerosol-generating device is a portable or handheld aerosol-generating device that is comfortable for a user to hold between the fingers of a single hand.

The aerosol-generating device may be substantially cylindrical in shape

The aerosol-generating device may have a length of between approximately 70 millimetres and approximately 120 millimetres.

The device may comprise a power supply for supplying electrical power to the electric heating element. The power supply may be any suitable power supply, for example a DC voltage source such as a battery. In one embodiment, the power supply is a Lithium-ion battery. Alternatively, the power supply may be a Nickel-metal hydride battery, a Nickel cadmium battery, or a Lithium based battery, for example a Lithium-Cobalt, a Lithium-Iron-Phosphate, Lithium Titanate or a Lithium-Polymer battery.

The device may comprise electric circuitry for controlling the supply of power from the power supply to the electric heating element. The electric circuitry may comprise one or more microprocessors or microcontrollers.

As used herein, the terms 'upstream' and 'downstream' are used to describe the relative positions of elements, or portions of elements, of the heat diffuser, aerosol-generating article, or aerosol-generating device, in relation to the direction in which air is drawn through the system during use thereof.

As used herein, the term 'longitudinal' is used to describe the direction between the upstream end and the downstream end of the heat diffuser, aerosol-generating article, or aerosol-generating device and the term 'transverse' is used to describe the direction perpendicular to the longitudinal direction.

As used herein, the term 'diameter' is used to describe the maximum dimension in the transverse direction of the heat diffuser, aerosol-generating article, or aerosol-generating device. As used herein, the term 'length' is used to describe the maximum dimension in the longitudinal direction.

There is provided a heated aerosol-generating article. The aerosol-generating article can be used together with an aerosol-generating device described above, or with an aerosol generating device according to the second or third aspects of the invention. The aerosol-generating article can be also used with other aerosol generating devices (not described in the present document).

The heated aerosol-generating article may be referred to herein as the article. In one embodiment, the article comprises a plurality of components which are coaxially aligned and assembled within a wrapper. The article has a mouth end and a distal end upstream from the mouth end. The article includes a liquid aerosol-forming substrate and a liquid retention medium for retaining the liquid aerosol-forming substrate. The wrapper may be formed from a sheet of liquid-impervious material.

Heated aerosol-generating articles that use a solid aerosol-forming substrate and that are formed in the shape of a rod are known. The wrapper used to assemble or circumscribe the components of such articles is typically a traditional porous cigarette paper. The use of a liquid aerosol-forming substrate may provide certain benefits over the use of a solid aerosol-forming substrate. One such benefit is the greater variety of flavours that may be provided in liquid substrate formulations compared to solid substrate formulations. Another benefit may be the lower environmental odour associated with the vaporisation of liquid substrates. However, it is desirable to provide a liquid aerosol-forming substrate in a traditional consumable design that has a cigarette look and feel. The use of an article that has a plurality of components including a liquid retention medium for retaining a liquid aerosol-forming substrate, and which is assembled within a liquid-impervious wrapper allows benefits of an e-cigarette type system with the look, feel, and convenience of a heated tobacco system.

In preferred embodiments the wrapper is a sheet of polymeric material, a sheet of treated paper, for example a polymer coated paper or a polymer impregnated paper, or a sheet of metallic foil. The wrapper may be a sheet of hydrophobic material such as a wax coated paper. A preferred liquid impervious wrapper may be formed from, or comprise, an aluminium laminate paper, a class of paper that is well known in the confectionary industry and as an inner liner in tobacco boxes. The liquid impervious wrapper may be referred to as a liquid impermeable wrapper.

In one embodiment, the article is preferably configured to be suitable for use with an electrically-operated aerosol-generating device. The article is preferably a consumable article having a mouth end for insertion into a user's mouth during use, a distal end upstream from the mouth end, and a mid-point located an equal distance between the mouth end and the distal end. The article comprises a liquid retention medium, at least a portion of which is located between the distal end and the mid-point.

In one embodiment, the article comprises a liquid aerosol-forming substrate contained within a frangible capsule, the frangible capsule being preferably located between the distal end and the mid-point. The article is configured such that, during use, air can be drawn through the article from the distal end to the mouth end.

Preferably, the liquid aerosol-forming substrate is releasably contained within the frangible capsule and the liquid retention medium is located in proximity to the frangible capsule for retaining the liquid aerosol-forming substrate within the article after its release from the frangible capsule.

The liquid aerosol-forming substrate may, alternatively, be incorporated into the liquid retention medium immediately prior to use. For example, a dose of liquid aerosol-forming substrate may be injected into the liquid retention medium immediately prior to use.

In one embodiment, the article comprises a first volatile liquid substrate contained within a first frangible capsule, and a second volatile liquid substrate contained within a second frangible capsule. The first and second frangible capsules are preferably located between the distal end and the mid-point. The article is configured such that, during use, air can be drawn through the article from the distal end to the mouth end.

Preferably, the first volatile liquid substrate is releasably contained within the first frangible capsule and the liquid retention medium is located in proximity to the first frangible capsule for retaining the first volatile liquid substrate within the article after its release from the first frangible capsule.Preferably, the second volatile liquid substrate is releasably contained within the second frangible capsule and the liquid retention medium is located in proximity to the second frangible capsule for retaining the second volatile liquid substrate within the article after its release from the second frangible capsule.

The article may comprise a liquid aerosol-forming substrate. The first volatile liquid substrate may be a liquid aerosol-forming substrate or may be a constituent part of a liquid aerosol-forming substrate, such as a liquid aerosol former or a nicotine source. The second volatile liquid substrate may also be a liquid aerosol-forming substrate or may be a constituent part of a liquid aerosol-forming substrate, such as an aerosol former or a nicotine source.

Where the liquid first volatile substrate is a first liquid aerosol-forming substrate and the second liquid volatile substrate is a second liquid aerosol-forming substrate, the first liquid aerosol-forming substrate may have a different composition to the second liquid aerosol-forming substrate. This may enable a user to have two different sensorial experiences using the same article. However, the first liquid aerosol-forming substrate may have the same composition as the second liquid aerosol-forming substrate.

Where the first volatile liquid substrate is a constituent part of a liquid aerosol-forming substrate and the second volatile liquid substrate is another constituent part of the aerosol-forming substrate, the first volatile substrate and the second volatile substrate combine to form the liquid aerosol-forming substrate.

The heated aerosol-generating article is a consumable that may be consumed by coupling or engaging with an aerosol-generating device, preferably an electrically-operated aerosol-generating device. The article is removably couplable with the aerosol-generating device. The article may be used once or twice and then disposed of. The number of preferred uses may depend on the number of frangible capsules provided. A method of using the heated aerosol-generating article may comprise the steps of coupling the article to an aerosol-generating device, activating a heating means of the aerosol-generating device, and drawing air through the heated aerosol-generating article. Liquid aerosol-forming substrate retained within the aerosol-generating article is then vaporised by heat energy supplied by the heating means and condenses to form an aerosol entrained in the air. When the use is over, for example when the aerosol-forming substrate has been consumed, the article may be removed from the device. A preferred method in one embodiment may comprise steps of releasing the liquid aerosol-forming substrate from a frangible capsule such that it is retained by the liquid retention medium of the article, coupling the article to an electrically-operated aerosol-generating device, activating a heating means of the electrically-operated aerosol-generating device, and drawing air through the article, the liquid aerosol-forming substrate being vaporised by heat energy supplied by the heating means and condensing to form an aerosol entrained in the air. In one embodiment, a preferred method may comprise steps of releasing at least one of the first volatile liquid substrate from a first frangible capsule and the second liquid volatile substrate from a second frangible capsule such that it is retained by the liquid retention medium of the article, coupling the article to an electrically-operated aerosol-generating device, activating a heating means of the electrically-operated aerosol-generating device, and drawing air through the article, the liquid retained by the liquid retention medium being vaporised by heat energy supplied by the heating means and condensing to form an aerosol entrained in the air.

Preferably, the heating means heats air that is drawn into the heated aerosol-generating device, the heated air passing over or through the liquid retention medium of the article to vaporise the liquid aerosol-forming substrate and allow formation of an aerosol. It is preferred that air is heated to a temperature of about 200°C to 220°C before passing over or through the liquid retention medium. Preferably, the air with entrained volatile components subsequently cools to a temperature of about 100°C within the article, allowing the volatile components to condense and form an aerosol. The heating means may alternatively heat the liquid retention medium by conduction or radiation in order to vaporise the liquid aerosol-forming substrate and allow formation of an aerosol.

Where the first liquid volatile substrate comprises a first liquid aerosol-forming substrate and the second liquid volatile substrate comprises a second liquid aerosol-forming substrate, one method of using the heated aerosol generating article comprises: releasing the first liquid aerosol-forming substrate from the first frangible capsule such that it is retained by the liquid retention medium; coupling the heated aerosol-generating article to an electrically-operated aerosol-generating device; activating a heating means of the electrically-operated aerosol-generating device; drawing air through the heated aerosol-generating device, the first liquid aerosol-forming substrate being vapourised by heat energy supplied by the heating means and condensing to form an aerosol entrained in the air; uncoupling the heated aerosol-generating article from the electrically operated aerosol-generating device; releasing the second liquid aerosol-forming substrate from the second frangible capsule such that it is retained by the liquid retention medium; coupling the heated aerosol-generating article to the electrically-operated aerosol-generating device; activating the heating means of the electrically-operated aerosol-generating device; and drawing air through the heated aerosol-generating device, the second liquid aerosol-forming substrate being vapourised by heat energy supplied by the heating means and condensing to form an aerosol entrained in the air.

Where the first liquid volatile substrate comprises a first constituent of a liquid aerosol-forming substrate and the second liquid volatile substrate comprises a second constituent of the liquid aerosol-forming substrate, one method of using the heated aerosol-generating article comprises: releasing the first liquid volatile substrate from the first frangible capsule such that it is retained by the liquid retention medium; releasing the second liquid volatile substrate from the second frangible capsule such that it is retained by the liquid retention medium and combines with the first liquid volatile substrate to form a liquid aerosol-forming substrate; coupling the heated aerosol-generating article to an electrically-operated aerosol-generating device; activating a heating means of the electrically-operated aerosol-generating device; and drawing air through the heated aerosol-generating device, the liquid aerosol-forming substrate being vapourised by heat energy supplied by the heating means and condensing to form an aerosol entrained in the air.

Preferably, the heating means heats air that is drawn into the heated aerosol-generating device, the heated air passing over or through the liquid retention medium to allow formation of an aerosol. However, the heating means may heat the liquid retention medium by conduction or radiation to allow formation of an aerosol.

As the article is intended to be disposed after one or two uses, the hygiene issues that may be associated with typical e-cigarette systems are overcome. Furthermore, the liquid aerosol-forming substrate does not directly contact a heating element and, thus, problems with overheating of the aerosol-forming substrate do not occur. The liquid impervious wrapper, if provided, helps to prevent leakage of liquid aerosol-forming substrate. Articles may be produced with a wide range of different liquid aerosol-forming substrate compositions, thereby providing the user with the wide range of flavours and experiences that can be provided by e-cigarette systems. In particular, where a first volatile liquid substrate comprises a first liquid aerosol-forming substrate and a second volatile liquid substrate comprises a second liquid aerosol-forming substrate, the article may be provided with multiple liquid aerosol-forming substrate compositions, and thereby provide the user with multiple flavours and experiences. In preferred embodiments, the articles may be consumed using aerosol-generating devices configured for heating aerosol-generating articles comprising solid aerosol-forming substrates. Thus, a user may select either a tobacco containing heated aerosol-generating article or select an article containing a liquid aerosol-forming substrate and consume either one using the same device.

As used herein, the term "volatile substrate" refers to an aerosol-forming substrate or a constituent part of an aerosol-forming substrate.

As used herein, the term "aerosol-forming substrate" refers to a substrate capable of releasing volatile compounds that can form an aerosol. An aerosol-forming substrate may be solid or liquid or comprise both solid and liquid components.

As used herein, the term "liquid aerosol-forming substrate" refers to an aerosol-forming substrate that is in a liquid rather than a solid form. A liquid aerosol-forming substrate may be at least partially absorbed by a liquid retention medium. A liquid-aerosol-forming substrate includes an aerosol-forming substrate in the form of a gel. Similarly, the term "liquid volatile substrate" refers to a substrate that is in a liquid rather than a solid form.

As used herein, the term "mouth end" refers to a portion of the heated aerosol-generating article where aerosol exits the article and is delivered into a user's mouth. In use, a user may draw on the mouth end of the article in order to inhale aerosol generated by the heated aerosol-generating article.

As used herein, the term "distal end" refers to an end of the article that opposes the mouth end.

As used herein, the terms "upstream" and "downstream" are used to describe the relative positions of components, or portions of components, of the heated aerosol-generating article in relation to the direction that air is drawn through the article or system during use. The mouth end of the article may also be referred to as the downstream end and the distal end of the article may also be referred to as the upstream end. Components, or portions of components, of the article may be described as being upstream or downstream of one another based on their relative positions between the mouth or downstream end and the distal or upstream end.

As used herein, the term "liquid-impervious" refers to the wrapper and indicates that aqueous liquids will not pass through the wrapper under typical operating conditions.

As used herein, the term 'sheet' denotes a laminar element having a width and length substantially greater than the thickness thereof. The width of a sheet is greater than 10 mm, preferably greater than 20 mm or 30 mm.

As used herein, the term 'longitudinal' is used to describe the direction between the upstream end and the downstream end of the aerosol-generating article or aerosol-generating device, and the term 'transverse' is used to describe the direction perpendicular to the longitudinal direction.

As used herein, the term 'diameter' is used to describe the maximum dimension in the transverse direction of the aerosol-generating article or aerosol-generating device. As used herein, the term 'length' is used to describe the maximum dimension in the longitudinal direction.

As used herein, the term "liquid retention medium" refers to a component that is capable of releasably retaining a liquid aerosol-forming substrate. The liquid retention medium may be, or may comprise, a porous or fibrous material that absorbs or otherwise retains a liquid aerosol-forming substrate that it is brought into contact with while allowing the liquid aerosol-forming substrate to be released by vaporisation.

As used herein, the term "frangible capsule" refers to a capsule that is capable of containing a liquid aerosol-forming substrate and releasing the liquid aerosol-forming substrate when broken or ruptured. The frangible capsule may be formed from, or comprise, a brittle material that is easily broken by a user to release its liquid aerosol-forming substrate contents. For example the capsule may be broken by external force such as finger pressure, or by contact with a piercing or rupturing element.

The heated aerosol-generating article may be substantially cylindrical in shape. The aerosol-generating article may be substantially elongate. The aerosol-generating article may have a length and a circumference substantially perpendicular to the length. The liquid retention medium may be substantially cylindrical in shape. The liquid retention medium may be substantially elongate. The liquid retention medium may also have a length and a circumference substantially perpendicular to the length.

The aerosol-generating article may have an external diameter of between approximately 5 millimetres and approximately 12 millimetres, for example of between approximately 6 millimetres and approximately 8 millimetres. In a preferred embodiment, the aerosol-generating article has an external diameter of 7.2 millimetres +/- 10 percent.

The aerosol-generating article may have a total length between approximately 25 mm and approximately 100 mm. The aerosol-generating article may have a total length between approximately 30 mm and approximately 100 mm. In one specific embodiment, the aerosol-generating article has a total length of approximately 45 mm. In another specific embodiment, the aerosol-generating article has a total length of approximately 33 mm.

The liquid retention medium may have a length of between about 7 mm and about 20 mm, for example between 8 mm and 15 mm. In one embodiment, the liquid retention medium may have a length of approximately 10 mm.

The heated aerosol-generating article may comprise a plurality of components assembled by, or circumscribed by, a wrapper in the form of a rod. For example, the article may comprise the liquid retention medium, and a mouthpiece located downstream of the liquid retention medium. The wrapper may comprise indicators, such as markings at positions along its length to indicate the location of the frangible capsule or of the first and second frangible capsules. The indicators may indicate to a user where to apply pressure to rupture or break the frangible capsule, or the first frangible capsule and the second frangible capsule respectively.

The mouthpiece may be located at the mouth end of the article. The mouthpiece may comprise a filter. The filter may be formed from one or more suitable filtration materials. Many such filtration materials are known in the art. In one embodiment, the mouthpiece may comprise a filter formed from cellulose acetate tow.

The article may comprise a porous or air-permeable plug located at the distal end of the article. Such a plug may act to help retain the liquid aerosol-forming substrate within the article. The plug may have an external diameter of a diameter of between approximately 5 millimetres and approximately 10 millimetres, for example of between approximately 6 millimetres and approximately 8 millimetres. In a preferred embodiment, the plug has an external diameter of 7.2 millimetres +/- 10%.

The plug may have a length of between approximately 2 millimetres and approximately 10 millimetres. For example, the mouthpiece may have a length of from about 3 mm to about 5 mm.

The article may comprise an aerosol-forming section or an aerosol-cooling section. The plurality of components may be co-axially aligned and assembled within the wrapper. The wrapper may be a traditional cigarette paper. The wrapper may be a polymeric film or a coated paper. The wrapper may be liquid-impervious.

The liquid retention medium preferably comprises an absorbent material, for example an absorbent polymeric material. Examples of suitable liquid retention materials include fibrous polymers and porous polymers such as open-cell foams. The liquid retention medium may comprise a fibrous cellulose acetate or a fibrous cellulose polymer. The liquid retention medium may comprise a porous polypropylene material. Suitable materials capable of retaining a liquid will be known to the skilled person.

The liquid retention medium is either located within an air-flow path through the heated aerosol-generating article or defines at least a portion of an air-flow path through the aerosol-generating article. Preferably, one or more holes defined through the liquid retention medium define a portion of the air-flow path through the heated aerosol-generating article between the distal end of the article and the mouth end of the article.

The liquid retention medium may be in the form of a tube having a central lumen. Walls of the tube would then be formed from, or comprise, a suitable liquid-retention material.

The heated aerosol-generating article may comprise a liquid aerosol-forming substrate contained within a frangible capsule. The heated aerosol-generating article may comprise liquid volatile substrates contained within frangible capsules. The frangible capsule or capsules are preferably spheroid, for example spherical or ovoid, having a maximum dimension of between 2 mm and 8 mm, for example between 4 mm and 6 mm. The frangible capsule or capsules may contain a volume of between 20 and 300 microlitres, for example between 30 and 200 microlitres. Such a range may provide between 10 and 150 puffs of aerosol to a user.

It is preferred that the liquid retention medium is capable of absorbing between 105% and 110% of the total volume of liquid contained within the frangible capsule. This helps to prevent leakage of liquid aerosol-forming substrate from the article after the frangible capsule has been broken to release its contents. It is preferred that the liquid retention medium is between 90% and 95% saturated after release of the liquid aerosol-forming substrate from the frangible capsule.

The frangible capsules may have a brittle shell, or may be shaped to facilitate rupture when subjected to external force. The frangible capsules may be configured to be ruptured by application of external force. For example, the frangible capsules may be configured to rupture at a specific defined external force, thereby releasing the liquid-aerosol-forming substrate. The frangible capsules may be configured with a weakened or brittle portion of its shell to facilitate rupture. The frangible capsules may be arranged for engagement with a piercing element for breaking the capsule and releasing the liquid aerosol-forming substrate. Preferably the frangible capsules have a burst strength of between about 0.5 and 2.5 kilograms force (kgf), for example between 1.0 and 2.0 kgf.

The shell of the frangible capsules may comprise a suitable polymeric material, for example a gelatin based material. The shell of the capsules may comprise a cellulose material or a starch material.

In one embodiment, the frangible capsule may be located adjacent to the liquid retention medium within the article such that the liquid-aerosol-forming substrate released from the frangible capsule can contact and be retained by the liquid retention medium. The frangible capsule may be located within the liquid retention medium. For example, the liquid retention medium may be in the form of a tube having a lumen and the frangible capsule containing the liquid aerosol-forming substrate may be located within the lumen of the tube.

In one embodiment, the frangible capsules may be located adjacent to the liquid retention medium within the article such that the liquid volatile substrate released from the frangible capsule can contact and be retained by the liquid retention medium. The frangible capsules may be located within the liquid retention medium. For example, the liquid retention medium may be in the form of a tube having a lumen and the frangible capsules containing the liquid volatile substrate may be located within the lumen of the tube.

Preferably, the heated aerosol-generating article comprises one or two frangible capsules comprising liquid volatile substrates. However, the heated aerosol-generating article may comprise any number of frangible capsules. The heated aerosol-generating article may comprise two or more frangible capsules comprising liquid volatile substrates.

The heated aerosol-generating article may comprise an aerosol generation section located downstream of the liquid retention medium. In use, the liquid aerosol-forming substrate is vaporised and volatile components of the substrate are drawn downstream from the liquid retention medium. The volatile components then cool in the aerosol forming section to form the inhalable aerosol. Preferably, the air with entrained volatile components cools to a temperature of about or below 100°C within the aerosol generation section. The aerosol forming section may be defined by a space within the article, or by the lumen of a tube within the article. The aerosol forming section may include an aerosol-cooling element, for example an aerosol-cooling element comprising a gathered sheet of polymeric material. A suitable aerosol-cooling element is described above.

As mentioned above, the article may comprise a liquid aerosol-forming substrate. In an embodiment with two capsules, the first volatile liquid substrate may be a liquid aerosol-forming substrate or may be a constituent part of a liquid aerosol-forming substrate, such as a liquid aerosol former or a nicotine source. The second volatile liquid substrate may also be a liquid aerosol-forming substrate or may be a constituent part of a liquid aerosol-forming substrate, such as an aerosol former or a nicotine source. Where the first volatile liquid substrate is a constituent part of a liquid aerosol-forming substrate, the second volatile liquid substrate is another constituent part of the aerosol-forming substrate and the combination of the first volatile substrate and the second volatile substrate forms the liquid aerosol-forming substrate.

The liquid aerosol-forming substrate comprises water. Preferably, the liquid aerosol-forming substrate also comprises an aerosol former such as propylene glycol or glycerine. The liquid aerosol-forming substrate preferably comprises a flavourant. The liquid aerosol-forming substrate may further comprise an active ingredient such as nicotine. Preferably the liquid-aerosol-forming substrate has a water content of between 10 and 25 weight percent, for example between 12 and 20 weight percent. Water is required to form a suitable inhalable aerosol. The liquid aerosol-forming substrate may comprise a nicotine solution. The liquid aerosol-forming substrate preferably comprises a tobacco-containing material comprising volatile tobacco flavour compounds which are released from the liquid upon heating. The liquid aerosol-forming substrate may comprise a non-tobacco material. The liquid aerosol-forming substrate may include solvents, ethanol, plant extracts and natural or artificial flavours.

As used herein, the term "aerosol former" refers to any suitable known compound or mixture of compounds that, in use, facilitates formation of a dense and stable aerosol. An aerosol former is substantially resistant to thermal degradation at the operating temperature of the aerosol-generating article. Suitable aerosol formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Preferred aerosol formers are polyhydric alcohols or mixtures thereof, such as triethylene glycol, 1,3-butanediol and, most preferred, glycerine.

In a preferable heated aerosol-generating system, an airflow path is defined when the heated aerosol-generating article is coupled to the aerosol-generating device. The air flow path include a point at which air enters the aerosol-generating device, a point at which air passes into the distal end of the heated aerosol-generating article, a point at which air passes over the liquid retention medium, and a point at which air passes out of the mouth end of the heated aerosol-generating article and into a user's mouth. In a preferable system, the aerosol-generating device acts to heat air at a point between entry into the aerosol-generating device and passing over the liquid retention medium. This allows heated air to vaporise a liquid aerosol-forming substrate retained by the liquid retention medium. Heating of the air may be accomplished by a heater such as a heating coil that is located within the airflow path and acts to directly heat the air prior to that air passing over the liquid retention medium.

The heated aerosol-generating system may comprise an air permeable heat accumulator or heat diffuser that is arranged in the air flow path to heat air. The term heat diffuser is used below. The heat diffuser may interact with a heater and take on heat energy. The heat energy is then passed to air passing through the heat diffuser. A heat diffuser is preferably a component having a high surface area and high porosity. Air should be able to flow through the heat diffuser without undergoing a significant pressure drop. Examples of suitable heat diffusers may be a porous metallic foam or a porous ceramic foam component arranged both in thermal contact with a heater and within the air flow path of the heated aerosol-generating system.

A heat diffuser may be a removable component of a heated aerosol-generating system. For example, heat diffuser may be in the form of a removably couplable component that engages with an aerosol generating device to alter the manner in which the aerosol generating device heats aerosol generating articles. As an example, an aerosol generating device may comprise an insertable heating element for insertion into a solid aerosol forming substrate of a heated aerosol-generating article. The heating element contacts the solid aerosol-forming substrate and heats it to generate an aerosol. A heat diffuser may be configured to engage with the insertable heating element. The heat diffuser may then be heated by the heating element and heat air that passes through the heating element. The heated air may then volatilize an aerosol-forming substrate of a heated aerosol-generating article that is located downstream of the heat diffuser. In this way the manner in which the aerosol-generating device heats an aerosol-forming substrate may be changed from direct contact to indirect heating of air. The same aerosol-generating device may then be used to heat different types of aerosol-generating article, thereby providing a greater choice to the user.

A preferred system may comprise a heated aerosol-generating device, at least one heated aerosol-generating article as described above having a liquid aerosol-forming substrate, and at least one heated aerosol-forming substrate having a solid aerosol-forming substrate, for example an aerosol-forming substrate made from homogenized tobacco material. Preferably, the system may further comprise a removably couplable heat diffuser for engagement with the aerosol-generating device to change the manner in which the aerosol-generating device provides heat to the aerosol-forming substrate.

Preferably, the aerosol-generating device is a portable or handheld aerosol-generating device that is comfortable for a user to hold between the fingers of a single hand.

The aerosol-generating device may be substantially cylindrical in shape

The aerosol-generating device may have a length of between approximately 70 millimetres and approximately 120 millimetres.

The device may comprise a power supply for supplying electrical power to the electric heating element. The power supply may be any suitable power supply, for example a DC voltage source such as a battery. In one embodiment, the power supply is a Lithium-ion battery. Alternatively, the power supply may be a Nickel-metal hydride battery, a Nickel cadmium battery, or a Lithium based battery, for example a Lithium-Cobalt, a Lithium-Iron-Phosphate, Lithium Titanate or a Lithium-Polymer battery.

The controller may be a simple switch. Alternatively the controller may be electric circuitry and may comprise one or more microprocessors or microcontrollers.

In a second aspect of the invention, a heated aerosol-generating article for use with an electrically-operated aerosol-generating device is provided. The heated aerosol-generating article is a consumable article having a mouth end for insertion into a user's mouth during use, a distal end upstream from the mouth end, and a mid-point located an equal distance between the mouth end and the distal end. The article comprises: a liquid aerosol-forming substrate contained within a frangible capsule, the frangible capsule being located between the distal end and the mid-point, and a liquid retention medium, at least a portion of which is located between the distal end and the mid-point, in which the article is configured such that, during use, air can be drawn through the article from the distal end to the mouth end.

In some embodiments of the heated aerosol-generating article according to the second aspect of the invention, one or more of the following features could be used:
- the liquid aerosol-forming substrate is releasably contained within the frangible capsule and the liquid retention medium is located in proximity to the frangible capsule for retaining the liquid aerosol-forming substrate within the article after its release from the frangible capsule;
- the heated aerosol-generating article comprises a plurality of components assembled by a wrapper in the form of a rod;
- the frangible capsule is located within the liquid retention medium in the heated aerosol-generating article;
- the liquid retention medium is in the form of a tube having a lumen and the frangible capsule is located within the lumen of the tube;
- the liquid retention means comprises an absorbent polymeric material;
- the frangible capsule is configured to be ruptured by application of external force;
- the frangible capsule is configured to be pierced by a piercing element;
- the heated aerosol-generating article comprises a cooling section located downstream from the liquid retention element;
- the liquid aerosol-forming substrate comprises between 10 weight percent and 25 weight percent water, an aerosol former, and at least one flavourant;
- the heated aerosol-generating article comprises a mouthpiece filter located at the mouth end of the article;
- the heated aerosol-generating article comprises a porous plug located at the distal end of the article;

In some embodiments of the heated aerosol-generating system, the system comprises a heated aerosol-generating article according to the second aspect of the invention, and an electrically-operated aerosol-generating device, the electrically-operated aerosol-generating device comprising means for heating the aerosol-forming substrate so as to form an aerosol. The heated aerosol-generating system may comprise a piercing element for piercing the frangible capsule.

A method of using a heated aerosol generating article according to the second aspect of the invention is also provided. The method comprises the steps of: releasing the liquid aerosol-forming substrate from the frangible capsule such that it is retained by the liquid retention medium; coupling the heated aerosol-generating article to an electrically-operated aerosol-generating device; activating a heating means of the electrically-operated aerosol-generating device; and drawing air through the heated aerosol-generating article, the liquid aerosol-forming substrate being vaporised by heat energy supplied by the heating means and condensing to form an aerosol entrained in the air. The heating means may heat air that is drawn into the heated aerosol-generating article, the heated air passing over or through the liquid retention medium to allow formation of an aerosol. The heating means may heat the liquid retention medium by conduction or radiation to allow formation of an aerosol.

In a third aspect of the inveniton, a heated aerosol-generating article comprising a plurality of components coaxially aligned and assembled within a wrapper is provided. The article has a mouth end and a distal end upstream from the mouth end, the article including: a liquid aerosol-forming substrate, and a liquid retention medium for retaining the liquid aerosol-forming substrate, in which the wrapper is formed from a sheet of liquid-impervious material.

In some embodiments of the heated aerosol-generating article according to the second aspect of the invention, one or more of the following features could be used:
- the liquid aerosol-forming substrate is releasably contained within a frangible capsule, the frangible capsule being located in proximity to the liquid retention medium for retaining the liquid aerosol-forming substrate within the article after its release from the frangible capsule;
- the frangible capsule is located within the liquid retention medium;
- the liquid retention medium is in the form of a tube having a lumen and the frangible capsule is located within the lumen of the tube;
- the liquid retention medium comprises an absorbent polymeric material;
- the frangible capsule is configured to be ruptured by application of external force;
- the frangible capsule is configured to be pierced by a piercing element;
- the heated aerosol-generating article comprises a cooling section located downstream from the liquid retention element;
- the liquid aerosol-forming substrate comprises between 10 weight percent and 25 weight percent water, an aerosol former, and at least one flavourant;
- the heated aerosol-generating article comprises a mouthpiece filter located at the mouth end of the article;
- the heated aerosol-generating article comprises a plug located at the distal end of the article, the article being configured such that air can be drawn into the article at or adjacent to the distal end, through the article, and out of the mouth end of the article;
- the wrapper is a sheet of polymeric material, a sheet of treated paper, or a sheet of metallic foil.

In some embodiments of the heated aerosol-generating system, the system comprises a heated aerosol-generating article according to the third aspect of the invention, and an electrically-operated aerosol-generating device, the electrically-operated aerosol-generating device comprising means for heating the aerosol-forming substrate so as to form an aerosol.

A method of using a heated aerosol generating article according to the third aspect of the invention is also provided. The method comprises the steps of: coupling the heated aerosol-generating article to an electrically-operated aerosol-generating device; activating a heating means of the electrically-operated aerosol-generating device; and drawing air through the heated aerosol-generating article, the liquid aerosol-forming substrate being vaporised by heat energy supplied by the heating means and condensing to form an aerosol entrained in the air. The liquid aerosol-forming substrate may be contained within a frangible capsule, and the method may further comprise the step of releasing the liquid aerosol-forming substrate from the frangible capsule such that it is retained within the article by the liquid retention medium.

In a fourth aspect of the present invention, a heated aerosol-generating article for use with an electrically-operated aerosol-generating device is provided. The heated aerosol-generating article is a consumable article having a mouth end for insertion into a user's mouth during use, a distal end upstream from the mouth end, and a mid-point located an equal distance between the mouth end and the distal end, the article comprising: a first volatile liquid substrate contained within a first frangible capsule, the frangible capsule being located between the distal end and the mid-point; a second volatile liquid substrate contained within a second frangible capsule, the second frangible capsule being located between the distal end and the mid-point; and a liquid retention medium, at least a portion of which is located between the distal end and the mid-point, in which the article is configured such that, during use, air can be drawn through the article from the distal end to the mouth end.

In some variants of the heated aerosol-generating article according to the fourth aspect of the invention, one or more of the following features could be used:
- the article comprises a liquid aerosol-forming substrate, the first volatile liquid substrate comprising a first constituent of the liquid aerosol-forming substrate and the second volatile liquid substrate comprising a second constituent of the liquid aerosol-forming substrate;
- the liquid aerosol-forming substrate comprises between 10 weight percent and 25 weight percent water, an aerosol former, and at least one flavourant;
- the first volatile liquid substrate comprises a first liquid aerosol-forming substrate and the second volatile substrate comprises a second liquid aerosol-forming substrate;
- the first volatile liquid substrate is releasably contained within the first frangible capsule and the liquid retention medium is located in proximity to the first frangible capsule for retaining the first volatile liquid substrate within the article after its release from the first frangible capsule, and the second volatile liquid substrate is releasably contained within the second frangible capsule and the liquid retention medium is located in proximity to the second frangible capsule for retaining the second volatile liquid substrate within the article after its release from the second frangible capsule;
- the heated aerosol-generating article comprises a plurality of components assembled by a wrapper in the form of a rod;
- the first and second frangible capsules are located within the liquid retention medium;
- the liquid retention medium is in the form of a tube having a lumen and the first and second frangible capsules are located within the lumen of the tube;
- the first and second frangible capsules are coaxially aligned in the lumen of the tube;
- the liquid retention medium comprises an absorbent polymeric material;
- at least one of the first and second frangible capsules is configured to be ruptured by application of external force;
- at least one of the first and second frangible capsules is configured to be pierced by a piercing element;
- the heated aerosol-generating article comprises a cooling section located downstream from the liquid retention medium;
- the heated aerosol-generating article comprises a mouthpiece filter located at the mouth end of the article;
- the heated aerosol-generating article comprises a porous plug located at the distal end of the article.

In some embodiments of the heated aerosol-generating system according to the fourth aspect of the invention, the system comprises a heated aerosol-generating article according to the embodiment or its variants as described above, and an electrically-operated aerosol-generating device, the electrically-operated aerosol-generating device comprising means for heating at least one of the first volatile liquid substrate and the second volatile liquid substrate retained in the liquid retention medium so as to form an aerosol. The heated aerosol-generating system may comprise a piercing element for piercing at least one of the frangible capsules.

According to a fifth aspect of the present invention, there is provided an electrically operated aerosol-generating device and a heat diffuser configured for use with an aerosol-generating article. The aerosol-generating article comprises an aerosol-forming substrate and has a mouth end and a distal end upstream from the mouth end. The electrically operated aerosol-generating device comprises an electric heating element and a housing having a cavity configured to receive the distal end of the aerosol-generating article. The heat diffuser is removably couplable to the aerosol-generating device and comprises a non-combustible porous body for absorbing heat from the electric heating element when the heat diffuser is coupled to the aerosol-generating device such that, in use, air drawn through the aerosol-generating article from the distal end to the mouth end is heated by the heat absorbed in the porous body.

According to a s aspect of the present invention, there is provided an electrically operated aerosol-generating device for an aerosol-generating system. The electrically operated aerosol-generating device comprises: a housing having a cavity configured to receive the distal end of an aerosol-generating article; an electric heating element; and a heat diffuser comprising a non-combustible, air permeable main body for absorbing and storing heat from the electric heating element such that, in use, air drawn through the main body of the heat diffuser is heated by heat stored in the main body.

The articles according to the second, third and fourth aspects of the invention can be used with the systems of first, fifth and sixth aspect of the invention.

Features described in relation to one or more aspects may equally be applied to other aspects of the invention.

The invention is further described, by way of example only, with reference to the accompanying drawings in which:
Figure 1 shows a schematic longitudinal cross-section of a heat diffuser of an aerosol-generating system according to a first embodiment of the present invention;
Figure 2a shows a schematic longitudinal cross-section of an aerosol-generating article in accordance with the first embodiment of the present invention which may be used with the heat diffuser of Figure 1;
Figure 2b-2h show schematic longitudinal cross-sections of aerosol-generating articles in accordance with various embodiments of the present invention which may be used with the heat diffuser of Figure 1;
Figure 3 shows a schematic view of the heat diffuser of Figure 1, the aerosol-generating article of Figure 2a and an aerosol-generating device according to the first embodiment of the present invention assembled for use;
Figure 4 shows a schematic longitudinal cross-section of an aerosol-generating article having a heat diffuser according to a second embodiment of the present invention;
Figure 5 shows the heated aerosol-generating article of Figures 2a to 2h according to the first embodiment of the invention, during use, engaged with an aerosol-generating device having external heating elements;
Figure 6 shows a heated aerosol-generating article of Figures 2a to 2h according to the first embodiment of the invention, during use, engaged with an aerosol-generating device having an internal heating element; and
Figure 7 shows a heated aerosol-generating article according to the first embodiment of the invention, during use, engaged with an aerosol-generating device having an internal heating element and a removably couplable heat diffuser.

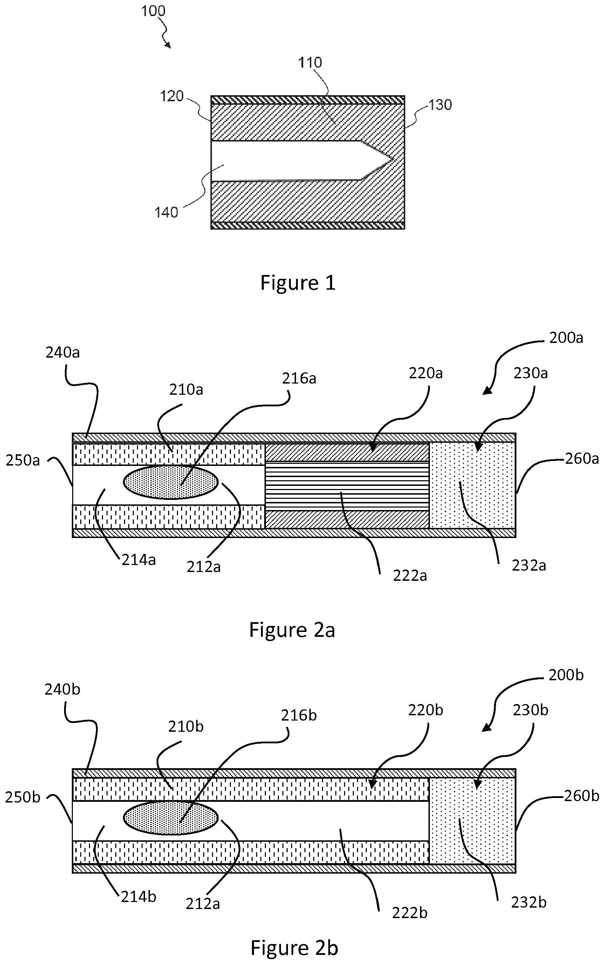

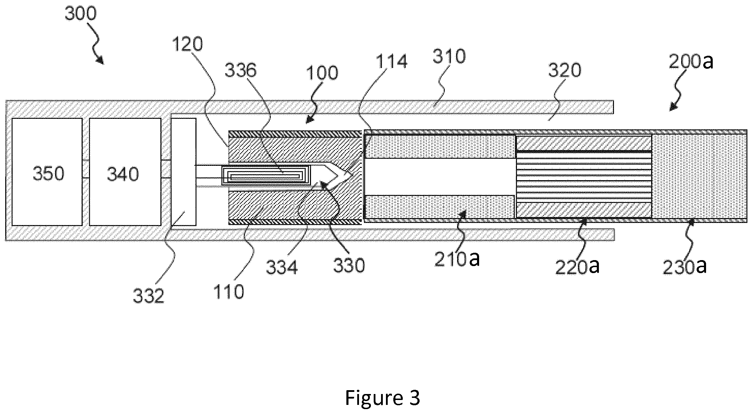

Figure 1 shows a heat diffuser 100 according to a first embodiment of the invention. The heat diffuser 100 includes a porous body 110 in the form of a cylindrical plug of thermally conductive material. The porous body 110 has an upstream or distal end 120 and a downstream or proximal end 130, opposite to the upstream end 120. A cavity in the form of a slot 140 is formed in the upstream end 120 of the porous body 110 and is arranged to receive a blade-shaped heating element, as discussed below in relation to Figure 3. The pores in the porous body 110 are interconnected to form a plurality of air flow passages extending through the porous body 110 from its upstream end 120 to its downstream end 130.

Figures 2a to 2h illustrate various embodiments of an aerosol-generating article. The articles shown in Figures 2a-h can be used with the aerosol-generating devices described here below, or with other aerosol-generating devices. The articles shown in Figures 2a-h can be used with the aerosol-generating systems described here below, or with other aerosol-generating systems.

Figure 2a illustrates an aerosol-generating article 200a for use with the heat diffuser 100 of Figure 1. An aerosol generating article can be also called a heated aerosol-generating article. The aerosol-generating article 200a comprises three elements arranged in coaxial alignment: a tubular liquid retention medium 210a, an aerosol-cooling element 220a, and a mouthpiece 230a. An aerosol cooling element can also be called an aerosol-generating section.. A mouthpiece can also be called a mouthpiece filter. Each of these three elements is a substantially cylindrical element, each having substantially the same diameter. These three elements are arranged co-axially and circumscribed by an outer wrapper 240a to form a cylindrical rod. These three elements are arranged sequentially and circumscribed by an outer wrapper 240a to form a cylindrical rod. The outer wrapper 240a may be non-porous. The outer wrapper 240a may be liquid-impervious.

The aerosol-generating article 200a has a distal or upstream end 250a and a proximal or mouth end 260a, opposite to the upstream end 250a, which a user inserts into his or her mouth during use. Once assembled, the total length of the aerosol-generating article 200a is about 33 mm about 45 mm and the diameter is about 7.2 mm.

The liquid retention medium 210a is located at the extreme distal or upstream end 250a of the aerosol-generating article 200a. In the embodiment illustrated in Figure 2a, the article 200a includes a frangible capsule 212a located within the lumen 214a of the liquid retention medium 210a. The frangible capsule 212a contains a liquid aerosol-forming substrate 216a.

The tubular liquid retention medium 210a has a length of 8 mm and is formed from fibrous cellulose acetate material. The liquid retention medium has a capacity to absorb 35 microlitres of liquid. The lumen 214a of the tubular liquid retention medium 210a provides an air flow path through the liquid retention medium 210a and also acts to locate the frangible capsule 212a. The material of the liquid retention medium may be any other suitable fibrous or porous material.

The frangible capsule 212a is shaped as an oval spheroid and has the long dimension of the oval aligned with the axis of the lumen 214a. The oval spheroid shape of the capsule may mean that it is easier to break than if it was circular spherical in shape, but other shapes of capsule may be used. The capsule 212a has an outer shell comprising a gelatin based polymeric material surrounding a liquid aerosol-forming substrate.

The liquid aerosol-forming substrate 216a comprises propylene glycol, nicotine extract, and 20 weight percent water. A wide range of flavourants may be optionally added. A wide range of aerosol formers may be used as alternative, or in addition to, propylene glycol. The capsule is about 4 mm in length and contains a volume of about 33 microlitres of liquid aerosol-forming substrate.

The aerosol-generating section may have a length of 18 mm. The aerosol-generating section may comprise a crimped and gathered sheet of polymeric material. The sheet of polymeric material is not densely packed and the aerosol-generating section does not cause significant pressure drop in air passing through the section. The crimped and gathered sheet of polymeric material may be termed an aerosol-cooling element and may have a length of about 18 mm, an outer diameter of about 7.12 mm, and an inner diameter of about 6.9 mm. In one embodiment, the aerosol-cooling element is formed from a sheet of polylactic acid having a thickness of 50 mm ± 2 mm. The sheet of polylactic acid has been crimped and gathered to define a plurality of channels that extend along the length of the aerosol-cooling element. The total surface area of the aerosol-cooling element is between 8000 mm² and 9000 mm², which is equivalent to approximately 500 mm² per mm length of the aerosol-cooling element. The specific surface area of the aerosol-cooling element is approximately 2.5 mm²/mg and it has a porosity of between 60% and 90% in the longitudinal direction. The polylactic acid is kept at a temperature of 160 degrees Celsius or less during use.

Porosity is defined herein as a measure of unfilled space in a rod including material such as an aerosol-cooling element. For example, if a diameter of the rod was 50% unfilled by the aerosol-cooling element, the porosity would be 50%. Likewise, a rod would have a porosity of 100% if the inner diameter was completely unfilled and a porosity of 0% if completely filled. The porosity may be calculated using known methods.

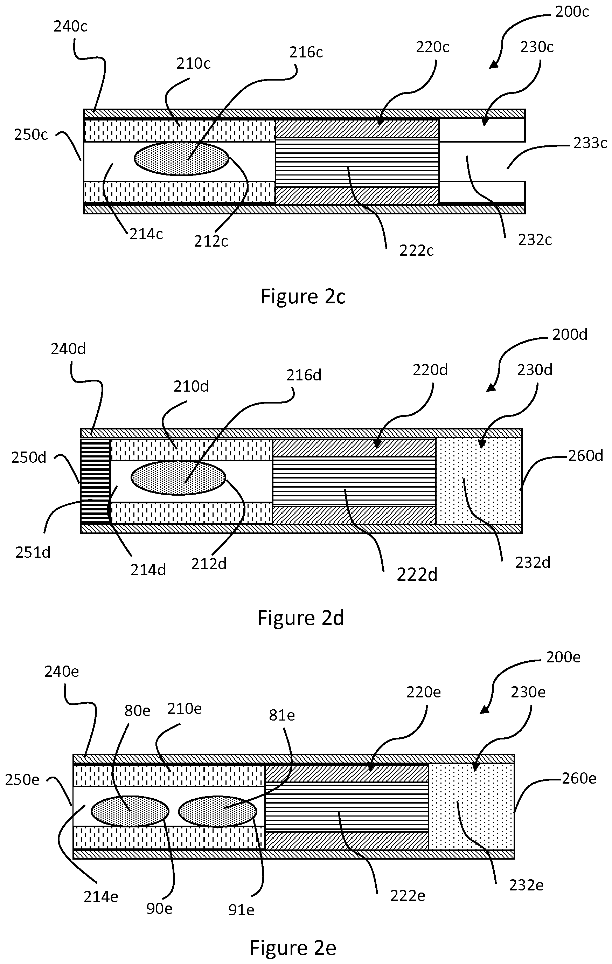

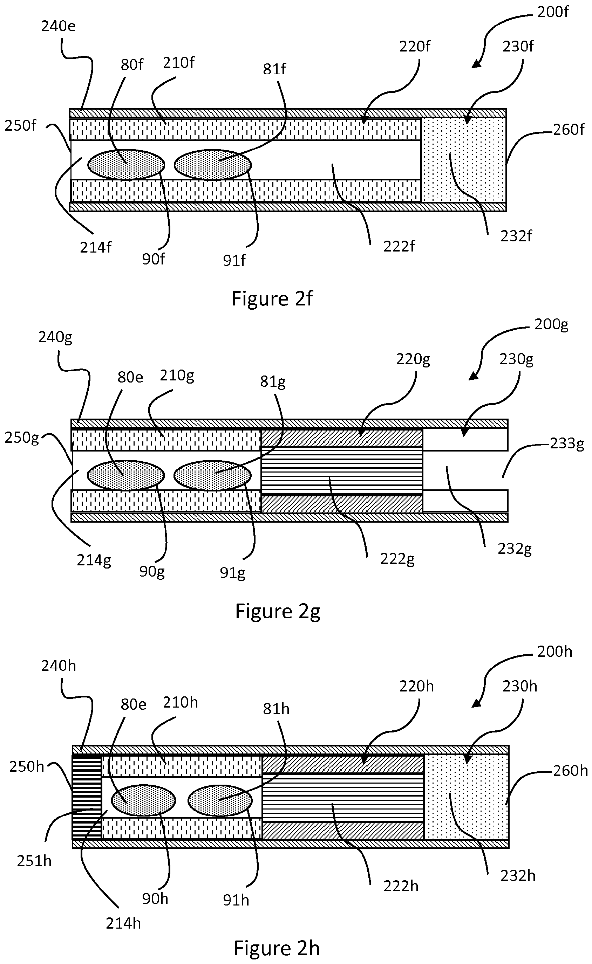

The aerosol-cooling element 220a is located immediately downstream of and abuts the liquid retention medium 210a. In use, volatile substances released from the aerosol-forming substrate 216a pass along the aerosol-cooling element 220a towards the mouth end 260a of the aerosol-generating article 200a. The volatile substances may cool within the aerosol-cooling element 220a to form an aerosol that is inhaled by the user. In the embodiment illustrated in Figure 2, the aerosol-cooling element 220a comprises a crimped and gathered sheet 222a of polylactic acid circumscribed by a wrapper 224a. The crimped and gathered sheet 222a of polylactic acid defines a plurality of longitudinal channels that extend along the length of the aerosol-cooling element 220a.

In alternative embodiments the aerosol-generating section may be a hollow section such as a hollow tube and may not comprise an aerosol-cooling element. Such embodiments are described below.

The mouthpiece 230a is located immediately downstream of and abuts the aerosol-cooling element 220a. In the embodiment illustrated in Figure 2, the mouthpiece 230a comprises a conventional cellulose acetate tow filter 232a of low filtration efficiency. The mouthpiece filter 230a may have a length of 7 mm and may be formed from cellulose acetate tow. Other suitable mouthpiece filters are known in the art.

To assemble the aerosol-generating article 200a, the three cylindrical elements described above are aligned and tightly wrapped within the outer wrapper 240a. In the embodiment illustrated in Figure 2a, the outer wrapper 240a is formed from a non-porous sheet material. The wrapper 240a may be a liquid-impervious wrapper, for example a wax-coated paper. Other suitable no-porous or liquid-impervious materials are known, for example polymeric films or hydrophobic papers. In other examples, the outer wrapper may comprise a porous material, such as cigarette paper.

Figures 2b to 2d illustrate alternative embodiments of heated aerosol-generating articles according to embodiments of the invention.

Figure 2b illustrates a heated aerosol-generating article 200b having a tubular liquid retention medium 210b and a mouthpiece filter 230b assembled in abutting relationship within a wrapper 224b. The wrapper 224b may be non-porous or liquid-impervious. A frangible capsule 212b containing a liquid aerosol-forming substrate is located within a lumen 214b of the liquid retention medium 210b. The overall length of the article 200b is 30 mm. The liquid retention medium has a length of 20 mm and the filter 230b has a length of 10 mm. The materials of the liquid retention medium, the frangible capsule, the liquid aerosol-forming substrate and the mouthpiece filter are the same as described above in relation to figure 2a.

Figure 2c illustrates a heated aerosol-generating article 200c that is identical to the article 200a described in relation to figure 2a with the difference that the cellulose acetate mouthpiece filter has been replaced by a rigid hollow mouthpiece 2303c. The mouthpiece 230c is tubular and has a lumen 233c defining an air flow path.

Figure 2d illustrates a heated aerosol-generating article 200d that is identical to the article 200a described in relation to figure 2a with the difference that a front-plug 251d is disposed at a distal end 250d of the article 200d. The front-plug 251d has a length of 3 mm and spans the distal end 250d of the article 200d. The front plug is formed of a highly porous cellulose acetate material and provides an air flow path into the article 200d. The front-plug 251 d may help to prevent egress of liquid aerosol-forming substrate during use of the article.

Figure 2e illustrates an embodiment of a heated aerosol-generating article 200e that is identical to the article 200a described in relation to figure 2a with the difference that a first volatile liquid substrate 80e is contained within a first frangible capsule 90e, and a second liquid volatile substrate 81e is contained within a second frangible capsule 91e. The first frangible capsule 90e is located within a lumen 214e of the tubular liquid retention medium 210e. The second frangible capsule 91e is also located within the lumen 214e of the tubular liquid retention medium 200e, and is arranged co-axially with the first frangible capsule 91 e within the lumen 214e such that the first frangible capsule 90e is arranged towards the distal end 250e and the second frangible capsule 91e is arranged towards the aerosol-generating section 220e.

The first frangible capsule 90e is shaped as an oval spheroid and has the long dimension of the oval aligned with the axis of the lumen. The oval spheroid shape of the first frangible capsule may mean that it is easier to break than if it was circular spherical in shape, but other shapes of capsule may be used. The first frangible capsule has an outer shell comprising a gelatin based polymeric material surrounding a liquid aerosol-forming substrate.

The second frangible capsule 91e may be identical to the first frangible capsule. The second frangible capsule 91 e may be also of different material or shape than the first frangible capsule 90e. The first frangible capsule 90e or the second frangible capsule 91e or both can have a different shape or a different construction from that described above.

The first volatile liquid substrate 80e comprises propylene glycol, nicotine extract, and 20 weight percent water. A wide range of flavourants may be optionally added. A wide range of aerosol-formers may be used as alternative, or in addition to, propylene glycol. The capsule 90e a long axis that is about 4 mm in length and contains a volume of about 33 microlitres of the first liquid aerosol-forming substrate.

The second volatile liquid substrate 81e also comprises propylene glycol, nicotine extract, and 20 weight percent water, but comprises a different flavourants to the first volatile liquid substrate 80e. The second frangible capsule 91e has a long axis that is about 4 mm in length and contains a volume of about 33 microlitres of the second liquid aerosol-forming substrate.

Figure 2f illustrates a heated aerosol-generating article 200f having a tubular liquid retention medium 210f and a mouthpiece filter 230f assembled in abutting relationship within a non-porous wrapper 240f. A first frangible capsule 90f containing a liquid aerosol-forming substrate is located within a lumen 214f of the liquid retention medium 210f. A second frangible capsule 91f containing the liquid aerosol-forming substrate is also located within the lumen 214f of the liquid retention medium 210f. The overall length of the article 200f is 30 mm. The liquid retention medium has a length of 20 mm and the filter 230f has a length of 10 mm. The materials of the liquid retention medium, the first and second frangible capsules, and the mouthpiece filter are the same as described above in relation to figures 2a and 2e. The liquid aerosol-forming substrate comprises propylene glycol, nicotine extract, 20 weight percent water and various flavourants.

Figure 2g illustrates a heated aerosol-generating article 200g that is identical to the article 200e described in relation to figure 2e with the difference that the cellulose acetate mouthpiece filter has been replaced by a rigid hollow mouthpiece 230g. The mouthpiece 230g is tubular and has a lumen 233g defining an air flow path.

Figure 2h illustrates a heated aerosol-generating article 200h that is identical to the article 200a described in relation to figure 2e with the difference that a front-plug 251h is disposed at a distal end 251h of the article 200h. The front-plug 251h has a length of 3 mm and spans the distal end 250h of the article 200h. The front plug is formed of a highly porous cellulose acetate material and provides an airflow path into the article 200h. The front-plug 251h may help to prevent egress of liquid aerosol-forming substrate during use of the article.

Figure 3 shows an aerosol-generating system in accordance with an embodiment of the present invention. The aerosol-generating system comprises the heat diffuser 100, the aerosol-generating article 200a, and an aerosol-generating device 300. In some embodiments, the aerosol generating system may comprise any of the aerosol-generating articles 200b-h. The system will now be described in connection with the aerosol-generating article 200a.

The aerosol-generating device includes a housing 310 defining a cavity 320 for receiving the heat diffuser 100 and the aerosol-generating article 200a. The device 300 further includes a heater 330 comprising a base portion 332 and a heating element in the form of a heater blade 334 that penetrates the heat diffuser 100 so that a portion of the heater blade 334 extends into the slot 140 in the porous body 110 when the heat diffuser 100 is received in the cavity 320, as shown in Figure 3. The heater blade 334 comprises resistive heating tracks 336 for resistively heating the heat diffuser 100. A controller 340 controls the operation of the device 300, including the supply of electrical current from a battery 350 to the resistive heating tracks 336 of the heater blade 334.

In the example shown in Figure 3, the frangible capsule has been ruptured prior to insertion of the article 200a into the cavity 320 of the device 300. Thus, the liquid aerosol-forming substrate is shown as being absorbed into the liquid retention medium 210a. In other examples, the frangible capsule may be ruptured following or during insertion of the aerosol-generating article 200a into the cavity 320 of the device 300. For example, the heat diffuser 100 may have a piercing member at its downstream end which is arranged to engage with and rupture the frangible capsule during insertion of the aerosol-generating article 200a into the cavity 320.

During use, the controller 340 supplies electrical current from the battery 350 to the resistive heating tracks 336 to heat the heater blade 334. Thermal energy is then absorbed by the porous body 110 of the heat diffuser 100 to heat the porous body 110. Air is drawn into the device 300 through air inlets (not shown) and subsequently through the heat diffuser 100 and along the aerosol-generating article 200a by a user from the distal end 120 of the heat diffuser 100 to the mouth end 260a of the aerosol-generating article 200a. As air is drawn through the porous body 110, the air is heated by the heat absorbed by the porous body 110 from the heater blade 334 before passing through the liquid retention medium 210a of the aerosol-generating article 200a to heat the liquid aerosol-forming substrate in the liquid retention medium 210a.

During the heating cycle, at least some of the one or more volatile compounds within the aerosol-generating substrate are evaporated. The vaporised aerosol-forming substrate is entrained in the air flowing through the liquid retention medium 210a and condenses within the aerosol-cooling element 220a and the mouthpiece portion 230a to form an inhalable aerosol, which exits the aerosol-generating article 200a at its mouth end 260a.

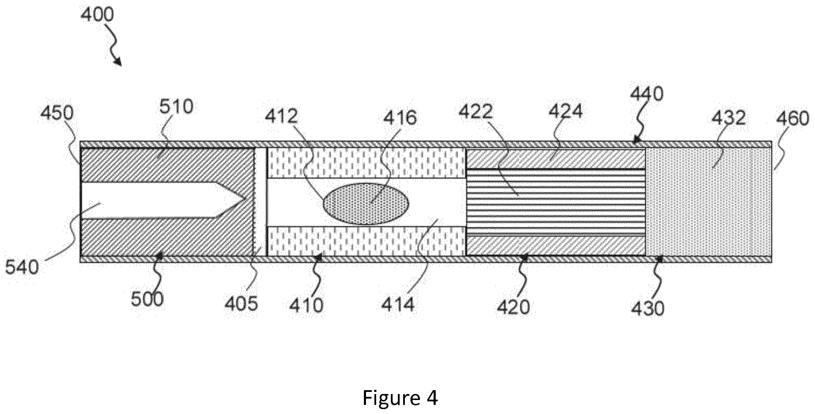

Figure 4 shows an aerosol-generating article 400 according to the present invention. The aerosol-generating article 400 has a similar structure to the aerosol-generating article 200a of Figure 2a and where the same features are present like reference numerals have been used. As with the aerosol-generating article 200a of Figure 2a, the aerosol-generating article 400 comprises liquid retention medium 410, an aerosol-cooling element 420, and a mouthpiece 430 arranged in coaxial alignment and circumscribed by a non-porous outer wrapper 440 to form a cylindrical rod. However, unlike the aerosol-generating article 200a of Figure 2a, in the aerosol-generating article 400 comprises a heat diffuser 500 at the upstream end 450 of the aerosol-generating article 400. The heat diffuser 500 comprises a porous body 510 in the form of a cylindrical plug of heat storage material, such as ceramic foam. The heat diffuser 500 is also circumscribed by the outer wrapper 440, such that the heat diffuser 500 forms part of the aerosol-generating article 400. As shown in Figure 4, a separation 405 is provided between the downstream end of the heat diffuser 500 and the upstream end of the liquid retention medium 410 to minimize the extent to which the liquid retention medium 410 might be heated by conduction from the heat diffuser 500.

As the heat diffuser 500 forms part of the aerosol-generating article 400, the heat diffuser 500 is removably coupled to the device as one with the rest of the aerosol-generating article 400, rather than as two separate components as is the case with the embodiments shown in Figures 1 to 3. Use of the aerosol-generating article 400 is otherwise the same as discussed above in relation to Figure 3.

The heat diffuser 500 could be also combined with any of the embodiments of the aerosol-generating article shown on Figures 2b-h (instead of the features of the an aerosol-generating article of Figure 2a).

Although the examples shown in Figures 1 to 4 illustrate that the aerosol-generating articles 200a-h and 400 include one or more frangible capsules, in other examples, three or more frangible capsules may be provided. In some embodiments, the articles may comprise a solid aerosol-forming substrate in addition to or instead of the frangible capsules.

Furthermore, although the examples shown in Figures 1 to 4 illustrate the heating element as a heating blade arranged to extend into the heat diffuser, the heating element may be provided as one or more heating elements extending around the periphery of the cavity. Additionally or alternatively, the heating element may comprise a susceptor located within the heat diffuser. For example, a blade-shaped susceptor may be located within the heat diffuser, in contact with the porous body. One or both ends of the susceptor may be sharpened or pointed to facilitate insertion into the heat diffuser.

Methods of using a heated aerosol-generating article according to embodiments of the invention using the aerosol-generating article shown in Figures 2a-d will be described with relation to the embodiment of figure 2a. The heated aerosol-generating articles disclosed herein are intended to be consumable items that are to be engaged with a separate aerosol-generating device for consumption.

Aerosol-generating devices for heating heated aerosol-generating articles having a solid aerosol-forming substrate are known in the art. In all embodiments described below, the first step is to release the liquid aerosol-forming substrate 216a from its frangible capsule 212a. This is achieved by squeezing the article in the region of the capsule between forefinger and thumb to apply an external force to rupture the frangible capsule. Once ruptured, the liquid aerosol-forming substrate is released onto and rapidly absorbed by the liquid retention medium. The article 200a is thus primed and ready for engagement with an aerosol-generating device.

Methods of using a heated aerosol-generating article according to embodiments of the invention using the aerosol-generating article shown in Figures 2e-h will be described with relation to the embodiment of figure 2d. The heated aerosol-generating articles disclosed herein are intended to be consumable items that are to be engaged with a separate aerosol-generating device for consumption.

Aerosol-generating devices for heating heated aerosol-generating articles having a solid aerosol-forming substrate are known in the art. In all embodiments described below, the first step is to release the first volatile liquid substrate 80e from the first frangible capsule 90e. This is achieved by squeezing the article in the region of the first frangible 90e capsule between forefinger and thumb to apply an external force to rupture the first frangible capsule 90e Once ruptured, the first liquid volatile substrate 80e is released onto and rapidly absorbed by the liquid retention medium.

Where the first liquid volatile substrate 80e is a first liquid aerosol-forming substrate, the article 200e is thus primed and ready for engagement with an aerosol-generating device. After a first use of the aerosol-generating article 200e, the second liquid volatile substrate 81e may be released from the second capsule 91e by squeezing the article in the region of the second capsule. The article 200e is thus primed and ready for engagement with an aerosol-generating device again for a second use of the article.

Where the first liquid volatile substrate 80e is a constituent of a liquid aerosol-forming substrate and the second liquid volatile substrate 81e is another constituent of the liquid aerosol-forming substrate, the second volatile liquid substrate 81e should also be released from the second frangible capsule 91e before the article 200e is primed and ready for engagement with an aerosol-generating device. Releasing the second volatile liquid substrate 81e from the second frangible capsule 91e causes the second volatile liquid substrate 81 e to mix with the first volatile liquid substrate 80e in the liquid retention medium to form the liquid aerosol-forming substrate. This is achieved by squeezing the article in the region of the second frangible capsule 91 e between forefinger and thumb to apply an external force to rupture the second frangible capsule 91e. Once ruptured, the second liquid volatile substrate 81e is released onto and rapidly absorbed by the liquid retention medium where it mixes with the first liquid volatile substrate 80e to form the liquid aerosol-forming substrate.

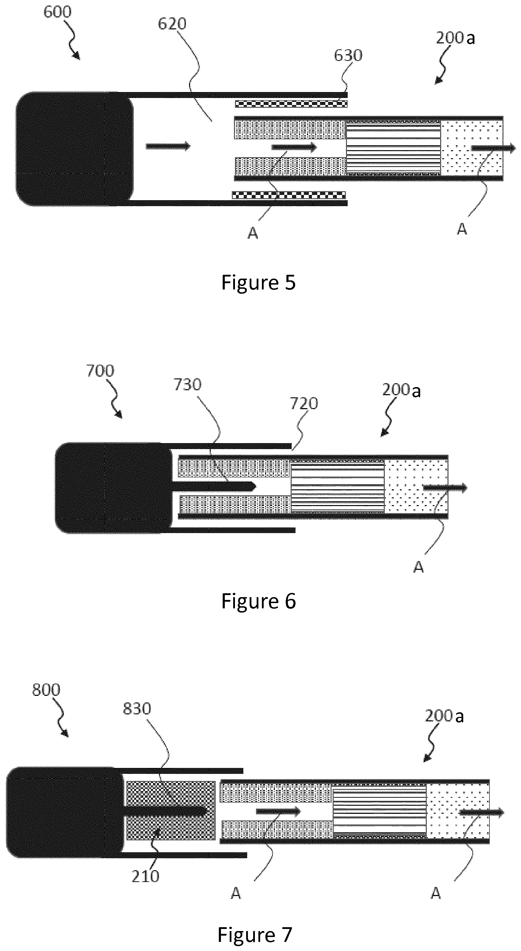

Figure 5 illustrates the use of an aerosol-generating device having external heating elements. Such an aerosol-generating device is known in the art for the consumption of aerosol-generating articles having solid aerosol-forming substrates. It will be described with reference to Figure 2a. However, embodiments of Figures 2b-h can be used. The device 600 defines a cavity 620 for receiving a distal portion of the heated aerosol-generating article 200a. A plurality of external heating elements 630 are located in the cavity and, when the article 200a is engaged within the cavity 620 the heating elements 630 surround the liquid retention medium. The external heating elements may be activated and then heat the liquid retention medium by conduction. The external heating elements may be activated and then heat the liquid retention medium by radiation. The liquid aerosol-forming substrate retained within the liquid retention means is heated and volatilised. As a user draws on the mouth end 260a of the article 200a the volatilised aerosol-forming substrate is entrained in the air drawn into the cooling element 220a of the article 200a. The volatilised aerosol-forming substrate cools within the aerosol-cooling section and condenses to form an inhalable aerosol. The inhalable aerosol is then inhaled by the user. The airflow path is shown by the arrows A.

Figure 6 illustrates the use of an aerosol-generating device having an internal heating element. Such an aerosol-generating device is known in the art for the consumption of aerosol-generating articles having solid aerosol-forming substrates. The device 700 defines a cavity 720 for receiving a distal portion of the heated aerosol-generating article 200a. A blade shaped heating element 730 is located in the cavity and, when the article 200a is engaged within the cavity 720 the heating element 730 extends into the lumen of the liquid retention medium. The internal heating element may be activated and then heat the liquid retention medium by radiation. The liquid aerosol-forming substrate retained within the liquid retention means is heated and volatilised. As a user draws on the mouth end 260a of the article 200a the volatilised aerosol-forming substrate is entrained in the air drawn into the cooling element 220a of the article 200a. The volatilised aerosol-forming substrate cools within the aerosol-cooling section and condenses to form an inhalable aerosol. The inhalable aerosol is then inhaled by the user.

Figure 7 illustrates a preferred method of using the heated aerosol-generating article 200a-h. The aerosol-generating device is an aerosol-generating device having an internal heating element as described in relation to figure 6. The aerosol-generating device 800 is engaged with a heat diffuser element 100, as described in relation to figure 1, as well as the aerosol-generating article 200a. The heat diffuser element 100 is a substantially cylindrical element formed from glass fibre. The heat diffuser element may be made from other porous materials such as ceramic fibres, ceramic foams, or sintered metals. The heat diffuser element 100 defines one or more longitudinally extending slots that allow the heat diffuser element to be penetrated by the heating element 830 of the aerosol-generating device 800. The internal heating element may be activated and then heat the heat diffuser element 100 by conduction. The internal heating element may be activated and then heat the heat diffuser element 100 by radiation. As a user draws air through the system, the air passes through the heated heat diffusor 100 and is heated up. This heated air is then drawn into the aerosol-generating article and passes through the liquid retention medium. The liquid aerosol-forming substrate retained within the liquid retention means is heated by this heated air and volatilised. As a user continues to draw on the mouth end 260a of the article 200a, the volatilised aerosol-forming substrate is entrained in the air drawn into the cooling element 220a of the article 200a. The volatilised aerosol-forming substrate cools within the aerosol-cooling element and condenses to form an inhalable aerosol. The inhalable aerosol is then inhaled by the user.

The specific embodiments and examples described above illustrate but do not limit the invention. It is to be understood that other embodiments of the invention may be made and the specific embodiments and examples described herein are not exhaustive.

## Claims

1. An aerosol-generating system comprising:
an aerosol-generating article (200a) comprising an aerosol-forming substrate (210a), the aerosol-generating article having a mouth end (260a) for insertion into a user's mouth and a distal end (250a) upstream from the mouth end; and
an electrically operated aerosol-generating device (300) comprising:
a housing (310) having a cavity (320) configured to receive the distal end of the aerosol-generating article;
an electric heating element (330); and
a heat diffuser (100) comprising a non-combustible porous body for absorbing heat from the electric heating element such that, in use, air drawn through the aerosol-generating article from the distal end to the mouth end is heated by the heat absorbed in the porous body before passing through the aerosol-generating substrate,
wherein the heat diffuser is removably couplable to the aerosol-generating device.

2. An aerosol-generating system according to claim 1, wherein the system is configured such that:
when the heat diffuser is coupled to the aerosol-generating device, the heat diffuser absorbs heat from the electric heating element and air drawn through the aerosol-generating article from the distal end to the mouth end is heated by the heat absorbed in the porous body of the heat diffuser.

3. An aerosol-generating system according to claim 1, wherein the system is configured such that:
when the heat diffuser is not coupled to the aerosol-generating device, the aerosol-generating article absorbs heat from the electric heating element.

4. An aerosol-generating system according to claim 1-3, wherein the porous body of the heat diffuser is configured to be penetrated by the electric heating element when the heat diffuser is coupled to the aerosol-generating device.

5. An aerosol-generating system according to claim 1-3, wherein the porous body of the heat diffuser defines a cavity or hole for receiving the electric heating element when the heat diffuser is coupled to the aerosol-generating device.

6. An aerosol-generating system according to claim 1, wherein the electric heating element is coupled to the porous body of the heat diffuser.

7. An aerosol-generating system according to claim 6, wherein the electric heating element is embedded in the porous body of the heat diffuser.

8. An aerosol-generating system according to claim 6 or 7, wherein the electric heating element comprises a susceptor coupled to the porous body of the heat diffuser.

9. An aerosol-generating system according to claim 6, 7 or 8, wherein the aerosol-generating device is configured to removably receive the heat diffuser and the electric heating element.

10. An aerosol-generating system according to any preceding claim, wherein the aerosol-generating article further comprises:
a liquid retention medium downstream of the heat diffuser; and
a frangible capsule in contact with the liquid retention medium,
wherein the aerosol-forming substrate is a liquid aerosol-forming substrate contained within the frangible capsule.

11. An aerosol-generating system according to claim 10, wherein the frangible capsule of the aerosol-generating article is located within the liquid retention medium.

12. An aerosol-generating system according to any preceding claim, wherein the electrically operated aerosol-generating device further comprises:
a power supply for supplying electrical power to the electrical heating element; and
electrical circuitry for controlling the supply of electrical power from the power supply to the electrical heater.

## Patentansprüche

1. Aerosolerzeugungssystem, aufweisend:
einen aerosolerzeugenden Artikel (200a), der ein aerosolbildendes Substrat (210a) aufweist, wobei der aerosolerzeugende Artikel ein Mundende (260a) zum Einführen in den Mund eines Benutzers und ein distales Ende (250a) zuströmseitig von dem Mundende aufweist; und
eine elektrisch betriebene Aerosolerzeugungsvorrichtung (300), aufweisend:
ein Gehäuse (310) mit einem Hohlraum (320), der ausgelegt ist, das distale Ende des aerosolerzeugenden Artikels aufzunehmen;
ein elektrisches Heizelement (330); und
einen Wärmeverteiler (100), der einen nicht brennbaren porösen Körper zum Absorbieren von Wärme von dem elektrischen Heizelement aufweist, sodass beim Gebrauch Luft, die durch den aerosolerzeugenden Artikel von dem distalen Ende zum Mundende gezogen wird, durch die in dem porösen Körper absorbierte Wärme erwärmt wird, bevor sie durch das aerosolerzeugende Substrat hindurchgeht, wobei der Wärmeverteiler mit der Aerosolerzeugungsvorrichtung lösbar koppelbar ist.

2. Aerosolerzeugungssystem nach Anspruch 1, wobei das System derart ausgelegt ist, dass:
wenn der Wärmeverteiler mit der Aerosolerzeugungsvorrichtung gekoppelt ist, der Wärmeverteiler Wärme von dem elektrischen Heizelement absorbiert und Luft, die von dem distalen Ende zum Mundende durch den Aerosolerzeugungsartikel gezogen wird, durch die in dem porösen Körper des Wärmeverteilers absorbierte Wärme erwärmt wird.

3. Aerosolerzeugungssystem nach Anspruch 1, wobei das System derart ausgelegt ist, dass:
wenn der Wärmeverteiler nicht mit der Aerosolerzeugungsvorrichtung gekoppelt ist, der aerosolerzeugende Artikel Wärme von dem elektrischen Heizelement absorbiert.

4. Aerosolerzeugungssystem nach Anspruch 1 bis 3, wobei der poröse Körper des Wärmeverteilers ausgelegt ist, von dem elektrischen Heizelement durchdrungen zu werden, wenn der Wärmeverteiler mit der Aerosolerzeugungsvorrichtung gekoppelt wird.

5. Aerosolerzeugungsvorrichtung nach Anspruch 1 bis 3, wobei der poröse Körper des Wärmeverteilers einen Hohlraum oder ein Loch zur Aufnahme des elektrischen Heizelements definiert, wenn der Wärmeverteiler mit der Aerosolerzeugungsvorrichtung gekoppelt wird.

6. Aerosolerzeugungssystem nach Anspruch 1, wobei das elektrische Heizelement mit dem porösen Körper des Wärmeverteilers gekoppelt ist.

7. Aerosolerzeugungssystem nach Anspruch 6, wobei das elektrische Heizelement in den porösen Körper des Wärmeverteilers eingebettet ist.

8. Aerosolerzeugungssystem nach Anspruch 6 oder 7, wobei das elektrische Heizelement einen Suszeptor aufweist, der mit dem porösen Körper des Wärmeverteilers gekoppelt ist.

9. Aerosolerzeugungsvorrichtung nach Anspruch 6, 7 oder 8, wobei die Aerosolerzeugungsvorrichtung ausgelegt ist, den Wärmeverteiler und das elektrische Heizelement lösbar aufzunehmen.

10. Aerosolerzeugungssystem nach einem der vorstehenden Ansprüche, wobei der aerosolerzeugende Artikel weiter aufweist:
ein Flüssigkeitsrückhaltemedium nachgeschaltet des Wärmeverteilers; und
eine zerbrechliche Kapsel in Kontakt mit dem Flüssigkeitsrückhaltemedium,
wobei das aerosolbildende Substrat ein flüssiges aerosolbildendes Substrat ist, das in der zerbrechlichen Kapsel enthalten ist.

11. Aerosolerzeugungssystem nach Anspruch 10, wobei sich die zerbrechliche Kapsel des aerosolerzeugenden Artikels innerhalb des Flüssigkeitsrückhaltemediums befindet.

12. Aerosolerzeugungssystem nach einem der vorstehenden Ansprüche, wobei die elektrisch betriebene Aerosolerzeugungsvorrichtung ferner aufweist:
eine Stromversorgung zum Bereitstellen von elektrischem Strom an das elektrische Heizelement; und
elektrische Schaltungen zum Steuern des Bereitstellens von elektrischem Strom von der Stromversorgung an die elektrische Heizvorrichtung.

## Revendications

1. Système générateur d'aérosol, comprenant :
un article générateur d'aérosol (200a) comprenant un substrat formant aérosol (210a), l'article générateur d'aérosol ayant une extrémité buccale (260a) pour l'insertion dans la bouche d'un utilisateur et une extrémité distale (250a) en amont de l'extrémité buccale ; et
un dispositif générateur d'aérosol à fonctionnement électrique (300) comprenant :
un logement (310) ayant une cavité (320) configurée pour recevoir l'extrémité distale de l'article générateur d'aérosol ;
un élément de chauffage électrique (330) ; et
un diffuseur de chaleur (100) comprenant un corps poreux non combustible pour absorber la chaleur de l'élément de chauffage électrique de telle sorte que, lors de l'utilisation, l'air aspiré à travers l'article générateur d'aérosol de l'extrémité distale à l'extrémité buccale est chauffé par la chaleur absorbée dans le corps poreux avant de traverser le substrat générateur d'aérosol,
dans lequel le diffuseur de chaleur peut être couplé de manière amovible au dispositif générateur d'aérosol.

2. Système générateur d'aérosol selon la revendication 1, dans lequel le système est configuré de telle sorte que :
lorsque le diffuseur de chaleur est couplé au dispositif générateur d'aérosol, le diffuseur de chaleur absorbe la chaleur de l'élément de chauffage électrique et l'air aspiré à travers l'article générateur d'aérosol de l'extrémité distale à l'extrémité buccale est chauffé par la chaleur absorbée dans le corps poreux du diffuseur de chaleur.

3. Système générateur d'aérosol selon la revendication 1, dans lequel le système est configuré de telle sorte que :
lorsque le diffuseur de chaleur n'est pas couplé au dispositif générateur d'aérosol, l'article générateur d'aérosol absorbe la chaleur de l'élément de chauffage électrique.

4. Système générateur d'aérosol selon la revendication 1 à 3, dans lequel le corps poreux du diffuseur de chaleur est configuré pour être pénétré par l'élément de chauffage électrique lorsque le diffuseur de chaleur est couplé au dispositif générateur d'aérosol.

5. Système générateur d'aérosol selon la revendication 1 à 3, dans lequel le corps poreux du diffuseur de chaleur définit une cavité ou un trou pour recevoir l'élément de chauffage électrique lorsque le diffuseur de chaleur est couplé au dispositif générateur d'aérosol.

6. Système générateur d'aérosol selon la revendication 1, dans lequel l'élément de chauffage électrique est couplé au corps poreux du diffuseur de chaleur.

7. Système générateur d'aérosol selon la revendication 6, dans lequel l'élément de chauffage électrique est intégré dans le corps poreux du diffuseur de chaleur.

8. Système générateur d'aérosol selon la revendication 6 ou 7, dans lequel l'élément de chauffage électrique comprend un suscepteur couplé au corps poreux du diffuseur de chaleur.

9. Système générateur d'aérosol selon la revendication 6, 7 ou 8, dans lequel le dispositif générateur d'aérosol est configuré pour recevoir de manière amovible le diffuseur de chaleur et l'élément de chauffage électrique.

10. Système générateur d'aérosol selon l'une quelconque des revendications précédentes, dans lequel l'article générateur d'aérosol comprend en outre :
un milieu de rétention de liquide en aval du diffuseur de chaleur ; et
une capsule frangible en contact avec le milieu de rétention de liquide,
dans lequel le substrat formant aérosol est un substrat formant aérosol liquide contenu dans la capsule frangible.

11. Système générateur d'aérosol selon la revendication 10, dans lequel la capsule frangible de l'article générateur d'aérosol est située à l'intérieur du milieu de rétention de liquide.

12. Système générateur d'aérosol selon l'une quelconque des revendications précédentes, dans lequel le dispositif générateur d'aérosol à fonctionnement électrique comprend en outre :
une alimentation électrique pour fournir de l'énergie électrique à l'élément de chauffage électrique ; et
des circuits électriques pour contrôler l'alimentation en énergie électrique de l'alimentation électrique au dispositif de chauffage électrique.
